# EUROPÄISCHE PATENTSCHRIFT

(11) **EP 3 229 758 B1**
(45) Veröffentlichungstag und Bekanntmachung des Hinweises auf die Patenterteilung: **02.01.2019**
(21) Anmeldenummer: 15791552.1
(22) Anmeldetag: 02.11.2015
(51) Int. Cl.: B05B 9/00, A61K 8/92, A61K 8/04, A61Q 5/06, A61K 8/31, A61K 8/34

(54) **MITTEL UND VERFAHREN ZUR TEMPORÄREN VERFORMUNG KERATINHALTIGER FASERN**
AGENTS AND METHODS FOR THE TEMPORARY SHAPING OF KERATIN-CONTAINING FIBERS
AGENT ET PROCÉDÉ DE MISE EN EN FORME TEMPORAIRE DE FIBRES KÉRATINIQUES

(30) Priorität: 10.12.2014 DE 102014225432
(43) Veröffentlichungstag der Anmeldung: 18.10.2017
(73) Patentinhaber: Henkel AG & Co. KGaA, 40589 Düsseldorf (DE)
(72) Erfinder: FÖRSTER, Thomas, 40591 Düsseldorf (DE); BAYERSDÖRFER, Rolf, 22589 Hamburg (DE); KNAPPE, Thorsten, 22869 Schenefeld (DE)
(86) Internationale Anmeldenummer: PCT/EP2015/075379
(87) Internationale Veröffentlichungsnummer: WO 2016/091466

(56) Entgegenhaltungen:
- EP-A1- 0 686 388
- CA-A1- 2 571 285
- JP-A- 2007 319 234
- US-A- 3 372 840
- US-A- 5 885 561
- US-A1- 2002 074 349
- US-A1- 2002 079 377
- US-A1- 2004 065 683
- US-A1- 2008 152 610
- US-A1- 2009 061 004
- US-A1- 2010 269 844
- US-A1- 2013 018 333

## Beschreibung

Die Anmeldung betrifft das technische Fachgebiet der temporären Umformung keratinhaltiger Fasern, insbesondere menschlicher Haare. Gegenstand der Anmeldung sind spezifische haarkosmetische Formulierungen, welche sich zur Applikation auf keratinhaltige Fasern mittels eines Entspannungsverdampfungsverfahrens eignen. Darüber hinaus sind die Verwendung dieser haarkosmetischen Formulierungen in Vorrichtungen zur Entspannungsverdampfung und Verfahren zur temporären Umformung keratinhaltiger Fasern Gegenstand der vorliegenden Anmeldung

Eine ansprechend aussehende Frisur wird heute allgemein als unverzichtbarer Teil eines gepflegten Äußeren angesehen. Dabei gelten aufgrund von aktuellen Modeströmungen immer wieder Frisuren als chic, welche sich bei vielen Haartypen nur unter Verwendung festigender Wirkstoffe aufbauen bzw. für einen längeren Zeitraum bis hin zu mehreren Tagen aufrechterhalten lassen. Daher spielen Haarbehandlungsmittel, welche einer permanenten oder temporären Formgebung der Haare dienen, eine wichtige Rolle. Während bei der permanenten Umformung die chemische Struktur der keratinhaltigen Faser durch Reduktion und Oxidation modifiziert wird, finden solche Modifikationen der chemischen Struktur bei der temporären Umformung nicht statt. Entsprechende Mittel zur temporären Verformung enthalten als festigenden Wirkstoff üblicherweise synthetische Polymere und/oder Wachse.

Die wichtigste Eigenschaft eines Mittels zur temporären Verformung keratinhaltiger Fasern, im Folgenden auch Stylingmittel genannt, besteht darin, den behandelten Fasern in der neu modellierten Form - d.h. einer den Fasern aufgeprägten Form - einen möglichst starken Halt zu geben. Handelt es sich bei den keratinhaltigen Fasern um menschliche Haare, spricht man auch von starkem Frisurenhalt oder von hohen Haltegrad des Stylingmittels. Der Frisurenhalt wird im Wesentlichen durch die Art und Menge der eingesetzten festigenden Wirkstoffe bestimmt, wobei jedoch auch ein Einfluss der weiteren Bestandteile des Stylingmittels sowie der Applikationsform gegeben sein kann.

Im Bereich der temporären Verformung keratinhaltiger Fasern hat insbesondere die Sprühapplikation entsprechender kosmetischer Zubereitungen eine große Bedeutung, wobei die Zubereitungen in der Regel als Pumpsprays oder Aerosolsprays appliziert werden. Hierzu werden die kosmetischen Zubereitungen in einer Abgabevorrichtung konfektioniert, aus denen sie entweder mittels mechanischer Krafteinwirkung oder mit Hilfe eines Treibmittels über ein Ventil versprüht werden. Beide Methoden haben offensichtliche Nachteile. Während Pumpsprays sich in der Regel nicht für eine lang anhaltende gleichmäßige Sprühapplikation haarkosmetischer Zubereitungen eignen, basieren Aersolsprays auf dem Einsatz von Treibmitteln oder Treibgasen, die einerseits keine kosmetische Wirkung entfalten und von denen andererseits bei unsachgemäßer Handhabung eine Gefährdung der Verbraucher ausgehen kann.

Vor diesem Hintergrund besteht ein Bedarf nach alternativen Wegen zur Zerstäubung haarkosmetischer Zubereitungen. Als ein solches alternatives Sprühverfahren hat sich die Entspannungsverdampfung erwiesen. Bei diesem Verfahren, das beispielsweise in der internationalen Patentanmeldung WO 2001/83071 A1 (Henkel) beschrieben wird, wird eine flüssige oder pastöse lösungsmittelhaltige Zusammensetzung in einem abgeschlossenen Raum auf eine Temperatur erhitzt, die über dem Siedepunkt des Lösungsmittels liegt, wodurch in der Zusammensetzung ein Überdruck erzeugt wird. Bei Entspannung (Drosselung) des Drucks verdampft die Flüssigkeit und kann nachfolgend beispielsweise mittels einer geeigneten Düse zerstäubt werden.

Auch wenn die Entspannungsverdampfung also grundsätzlich für die Sprühapplikation haarkosmetischer Zubereitungen geeignet ist, so kann gleichzeitig doch nicht jede haarkosmetische Zubereitung mittels eines Entspannungsverdampfungsverfahrens zerstäubt werden. Dies liegt einerseits an der für die Entspannungsverdampfung notwendigen Erhitzung der kosmetischen Zubereitung, andererseits an den Spezifika der durch Entspannungsverdampfung erzeugten Sprühnebel, beispielsweise der erzeugten Tröpfchengröße und Tröpfchendichte im Sprühnebel. Aufgabe der vorliegenden Erfindung war es daher, spezifische haarkosmetische Zubereitungen zur temporären Verformung keratinhaltiger Fasern zur Verfügung zu stellen, welche sich aufgrund ihrer chemischen und physikalischen Eigenschaften zur zielgerichteten Sprühapplikation mittels einer Vorrichtung zur Entspannungsverdampfung eignen. Weiterhin sollen die Zubereitungen geeignet sein, nach einer Applikation mittels eines Entspannungsverfahrens einen hohen Haltegrad, insbesondere einen hohen Langzeithaltegrad, und einen hohen Volumeneffekt zu realisieren. Es hat sich gezeigt, dass zur Lösung dieser Aufgabe aus der Vielzahl bekannten haarkosmetisch wirksamen Stlyingzubereitungen insbesondere lösungsmittelhaltige Zubereitungen mit einem spezifischen Anteil hydrophober Wirkstoffe geeignet sind.

Gegenstand der vorliegenden Erfindung ist somit ein kosmetisches Produkt gemäss Anspruch 1, umfassend
a) eine kosmetische Zubereitung, enthaltend bezogen auf ihr Gesamtgewicht
   a1) 45 bis 95 Gew.-% mindestens eines polaren Lösungsmittels;
   a2) 1,0 bis 25 Gew.-% mindestens eines Fettstoffs;
b) eine Vorrichtung zur Entspannungsverdampfung der kosmetischen Zubereitung a).

Die kosmetische Zubereitung a) ist flüssig. Bevorzugte kosmetische Zubereitungen a) liegen in Form einer Emulsion vor.

Erfindungsgemäße kosmetische Zubereitungen sind über einen weiten Viskositätsbereich realisierbar. Beispielhaft Viskositäten sind
- 10.000 bis 80.000 mPas, vorzugsweise 20.000 bis 70.000 mPas (20°C, Brookfield Model D220, Spindel 5, 5rpm);
- 5.000 bis 30.000 mPas, vorzugsweise 7.000 bis 20.000 mPas (20°C, Brookfield Model D220, Spindel 6, 5rpm);
- 50.000 bis 300.000 mPas, vorzugsweise 70.000 bis 250.000 mPas (20°C, Brookfield Model D220, TE Helipath, 5rpm);
- 100.000 bis 1.200.000 mPas, vorzugsweise 150.000 bis 1.000.000 mPas (20°C, Brookfield Model D220, TE, 5rpm);
- 2.000.000 bis 4.000.000 mPas, vorzugsweise 2.500.000 bis 3.500.000 mPas (20°C, Brookfield Model D220, TE, 1rpm);
- 1.500.000 bis 4.000.000 mPas, vorzugsweise 2.000.000 bis 3.500.000 mPas (20°C, Brookfield Model D220, TF Helipath, 1rpm);
- 400.000 bis 2.000.000 mPas, vorzugsweise 500.000 bis 1.500.000 mPas (20°C, Brookfield Model D220, TF, 5rpm);
- 4.000.000 bis 10.000.000 mPas, vorzugsweise 4.500.000 bis 9.000.000 mPas (20°C, Brookfield Model D220, TF, 1rpm).

Die erfindungsgemäße kosmetische Zubereitung enthält als ersten wesentlichen Bestandteil 45 bis 95 Gew.-% mindestens eines polaren Lösungsmittels a1). Bevorzugte kosmetische Produkte sind dadurch gekennzeichnet, dass der Gewichtsanteil des polaren Lösungsmittels a1) am Gesamtgewicht der kosmetischen Zubereitung a) 60 bis 92 Gew.-%, vorzugsweise 70 bis 90 Gew.-% beträgt. Entsprechende Mittel zeichnen sich durch eine gute kosmetische Wirkung bei gleichzeitig guter Applizierbarkeit aus.

Zur Verbesserung der Anwendungseigenschaften erfindungsgemäßer kosmetischer Zubereitungen bei gleichzeitiger Minimierung der thermischen Belastung etwaiger Wirk- oder Hilfsstoffe im Verlauf des Entspannungsverdampfungsverahrens, hat es sich als vorteilhaft erwiesen, polare Lösungsmittel a1) einzusetzen, welche eine Siedetemperatur (20°C, 1013 mbar) zwischen 50 und 110°C, vorzugsweise zwischen 70 und 105°C aufweisen. Als besonders geeignet haben sich dabei Ethanol, Isopropanol, Gylcerin und Wasser erwiesen, welche aus diesem Grund als polare Lösungsmittel a1) bevorzugt werden.

Besonders bevorzugte polare Lösungsmittel a1) oder Lösungsmittelsysteme sind dadurch gekennzeichnet, dass
- der Gewichtsanteil von Wasser und Ethanol am Gesamtgewicht des polaren Lösungsmittels a1) vorzugsweise mindestens 60 Gew.-%, bevorzugt mindestens 70 Gew.-%, besonders bevorzugt mindestens 80 Gew.-% und insbesondere mindestens 90 Gew.-% beträgt;
- der Gewichtsanteil von Wasser am Gesamtgewicht des polaren Lösungsmittels a1) mehr als 70 Gew.-%, vorzugsweise mehr als 80 Gew.-% und insbesondere mehr als 90 Gew.-% beträgt.

Ein zweiter wesentlicher Bestandteil erfindungsgemäßer kosmetischer Zusammensetzungen ist der Fettstoff a2). In Bezug auf die Herstellbarkeit, Applizierbarkeit und kosmetische Wirkung erfindungsgemäßer kosmetischer Zusammensetzungen hat es sich als vorteilhaft erwiesen, wenn der Gewichtsanteil des Verdickers a2) am Gesamtgewicht der kosmetischen Zubereitung a) 2,0 bis 22 Gew.-%, vorzugsweise 4,0 bis 20 Gew.-% beträgt.

Als "Fettstoffe" werden in Wasser im Wesentlichen unlösliche hydrophobe Substanzen bezeichnet. Die Löslichkeit der Fettstoffe in Wasser (20°C) beträgt bevorzugt weniger als 5g pro 100 g Wasser, vorzugsweise weniger als 1g pro 100 g Wasser und insbesondere weniger als 0,1 g pro 100 g Wasser.

Die Fettstoffe können unter Normalbedingungen in fester oder flüssiger Form vorliegen. Eine erste Gruppe bevorzugter Fettstoffe a2) bilden die Wachse. Das Wachs a2) kann natürlichen oder synthetischen Ursprungs sein. Bevorzugte Wachse schmelzen oberhalb von 40°C, besonders bevorzugt oberhalb von 50°C, insbesondere bei Temperaturen zwischen 50°C und 90°C.

Als Wachse a2) können erfindungsgemäß feste Paraffine oder Isoparaffine, Pflanzenwachse wie Candelillawachs, Carnaubawachs, Espartograswachs, Japanwachs, Korkwachs, Zuckerrohrwachs, Ouricurywachs, Montanwachs, Sonnenblumenwachs, Fruchtwachse und tierische Wachse, wie z. B. Bienenwachse und andere Insektenwachse, Walrat, Schellackwachs, Wollwachs und Bürzelfett, weiterhin Mineralwachse, wie z. B. Ceresin und Ozokerit oder die petrochemischen Wachse, wie z. B. Petrolatum, Paraffinwachse, Microwachse aus Polyethylen oder Polypropylen und Polyethylenglycolwachse eingesetzt werden. Es kann vorteilhaft sein, hydrierte oder gehärtete Wachse einzusetzen. Weiterhin sind auch chemisch modifizierte Wachse, insbesondere die Hartwachse, z. B. Montanesterwachse, Sasolwachse und hydrierte Jojobawachse, einsetzbar.

Weiterhin geeignet sind die Triglyceride gesättigter und gegebenenfalls hydroxylierter C16-30-Fettsäuren, wie z. B. gehärtete Triglyceridfette (hydriertes Palmöl, hydriertes Kokosöl, hydriertes Rizinusöl), Glyceryltribehenat oder Glyceryltri-12-hydroxystearat, weiterhin synthetische Vollester aus Fettsäuren und Glykolen (z. B. Syncrowachs®) oder Polyolen mit 2-6 C-Atomen, Fettsäuremonoalkanolamide mit einem C12-22-Acylrest und einem C2-4-Alkanolrest, Ester aus gesättigten und/oder ungesättigten, verzweigten und/oder unverzweigten Alkancarbonsäuren einer Kettenlänge von 1 bis 80 C-Atomen und gesättigten und/oder ungesättigten, verzweigten und/oder unverzweigten Alkoholen einer Kettenlänge von 1 bis 80 C-Atomen, darunter z. B. synthetische Fettsäure-Fettalkoholester wie Stearylstearat oder Cetylpalmitat, Ester aus aromatischen Carbonsäuren, Dicarbonsäuren bzw. Hydroxycarbonsäuren (z. B. 12-Hydroxystearinsäure) und gesättigten und/oder ungesättigten, verzweigten und/oder unverzweigten Alkoholen einer Kettenlänge von 1 bis 80 C-Atomen, Lactide langkettiger Hydroxycarbonsäuren und Vollester aus Fettalkoholen und Di- und Tricarbonsäuren, z. B. Dicetylsuccinat oder Dicetyl-/stearyladipat, sowie Mischungen dieser Substanzen.

Die Wachskomponenten können auch aus der Gruppe der Ester aus gesättigten, unverzweigten Alkancarbonsäuren einer Kettenlänge von 14 bis 44 C-Atomen und gesättigten, unverzweigten Alkoholen einer Kettenlänge von 14 bis 44 C-Atomen ausgewählt werden, sofern die Wachskomponente oder die Gesamtheit der Wachskomponenten bei Raumtemperatur fest sind. Die Wachskomponenten können beispielsweise aus der Gruppe der C16-36-Alkylstearate, der C10-40-Alkylstearate, der C2-40-Alkylisostearate, der C20-40-Dialkylester von Dimersäuren, der C18-38-Alkylhydroxystearoylstearate, der C20-40-Alkylerucate ausgewählt werden, ferner sind C30-50-Alkylbienenwachs sowie Cetearylbehenat einsetzbar. Auch Silikonwachse, zum Beispiel Stearyltrimethylsilan/Stearylalkohol sind gegebenenfalls vorteilhaft. Bevorzugte Wachskomponenten sind die Ester aus gesättigten, einwertigen C20-C60-Alkoholen und gesättigten C8-C30-Monocarbonsäuren, insbesondere ein C20-C40-Alkylstearat bevorzugt, das unter dem Namen Kesterwachs® K82H von der Firma Koster Keunen Inc. erhältlich ist. Das Wachs oder die Wachskomponenten sollten bei 25 °C fest sein, jedoch im Bereich von 35-95 °C schmelzen, wobei ein Bereich von 45-85 ° C bevorzugt ist.

Weitere bevorzugte Wachskomponenten sind Fettalkohole. Als Fettalkohole können beispielsweise Stearylalkohol, Cetylalkohol, Laurylalkohol, Myristylalkohol, Arachidylalkohol, Caprylalkohol, Caprinalkohol und Behenylalkohol eingesetzt werden.

Bevorzugt ist das Wachs a2) aus Bienenwachs (Cera Alba), Carnaubawachs, Candelillawachs, Montanwachs, Cetylpalmitat, mikrokristallinen Wachsen (mikrokristalline Paraffine) und Gemischen davon ausgewählt. Besonders bevorzugt ist der Einsatz eines Fettstoffs a2) aus der Gruppe Bienenwachs (Cera Alba), Carnaubawachs und mikrokristallinen Wachsen (mikrokristalline Paraffine).

Natürliche, chemisch modifizierte und synthetische Wachse können alleine oder in Kombination eingesetzt werden. Die erfindungsgemäße Lehre umfasst somit auch den kombinierten Einsatz von mehreren Wachsen. Weiterhin sind auch eine Reihe von Wachsmischungen, ggf. in Abmischung mit weiteren Zusätzen, im Handel erhältlich. Die unter den Bezeichnungen "Spezialwachs 7686 OE" (eine Mischung aus Cetylpalmitat, Bienenwachs, mikrokristallinem Wachs und Polyethylen mit einem Schmelzbereich von 73-75 °C; Hersteller: Kahl & Co), Polywax® GP 200 (eine Mischung von Stearylalkohol und Polyethylenglykolstearat mit einem Schmelzpunkt von 47-51 deg. C; Hersteller: Croda) und "Weichceresin® FL 400" (ein Vaseline/Vaselinöl/Wachs-Gemisch mit einem Schmelzpunkt von 50-54 deg. C; Hersteller: Parafluid Mineralölgesellschaft) sind Beispiele für erfindungsgemäß bevorzugt eingesetzte Mischungen. Eine weitere besonders bevorzugte Mischung von Wachsen a2) umfasst Bienenwachs und Carnaubawachs, gegebenenfalls in Kombination mit mikrokristallinem Wachs.

Eine zweite Gruppe erfindungsgemäß bevorzugter Fettstoffe a2) bilden die Öle. Die Öle a2) können natürlichen oder synthetischen Ursprungs sein. Bevorzugte Öle schmelzen unterhalb von 10°C, besonders bevorzugt unterhalb von 0°C.

Bevorzugte erfindungsgemäße kosmetische Zubereitungen a) enthalten mindestens ein Öl aus der Gruppe der Silikonöle. Zur Gruppe der Silikonöle zählen insbesondere die Dimethicone, zu welchen auch die Cyclomethicone zu rechnen sind, die aminofunktionellen Silikone sowie die Dimethiconole. Die Dimethicone können sowohl linear als auch verzweigt als auch cyclisch oder cyclisch und verzweigt sein. Geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate.

Weitere bevorzugte erfindungsgemäße kosmetische Zubereitungen a) enthalten mindestens ein Öl aus der Gruppe der Esteröle, das heißt Ester von C6-C30-Fettsäuren mit C2-C30-Fettalkoholen, vorzugsweise Monoester der Fettsäuren mit Alkoholen mit 2 bis 24 C-Atomen wie beispielsweise Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkohol-caprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V).

Die Gruppe der Fettstoffe a2) umfasst weiterhin die folgenden bevorzugten Substanzen:
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether.
- natürliche (pflanzliche) Öle können Triglyceride und Mischungen von Triglyceriden eingesetzt werden. Bevorzugte natürliche Öle sind Sojaöl, Rapsöl, Orangenöl, Kokosnussöl, (süßes) Mandelöl, Walnussöl, Pfirsichkernöl, Aprikosenkernöl, Avocadoöl, Teebaumöl (Tea Tree Oil), Sesamöl, Sonnenblumenöl, Tsubakiöl, Nachtkerzenöl, Reiskleieöl, Palmkernöl, Mangokernöl, Wiesenschaumkrautöl, Distelöl, Macadamianussöl, Traubenkernöl, Amaranthsamenöl, Arganöl, Bambusöl, Olivenöl, Weizenkeimöl, Kürbiskernöl, Malvenöl, Haselnussöl, Safloröl, Canolaöl, Sasanquaöl, Jojobaöl, Rambutanöl, Kakaoabutter und Shea-Butter.
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol® CC),
- Trifettsäureester von gesättigten und/oder ungesättigten linearen und/oder verzweigten Fettsäuren mit Glycerin,
- Polyolfettsäureester, wie beispielsweise das Handelsprodukt Hydagen® HSP (Cognis) oder Sovermol - Typen (Cognis), aus Fettsäure und/oder Fettsäurederivaten und Polyolen mit mindestens zwei Hydroxygruppen, und mit einer Kohlenstoffkette von 2 bis 30 Kohlenstoffatomen wie beispielsweise Fettsäureester von Trimethylolpropan.

Besonders bevorzugt ist der Einsatz eines Fettstoffs a2) aus der Gruppe der Silikonöle, Paraffinöle, pflanlichen Öle und Esteröle, vorzugsweise aus der Gruppe der Silikonöle, Paraffinöle und der pflanzlichen Öle.

Die erfindungsgemäßen kosmetischen Produkte umfassen neben der kosmetischen Zubereitung a) weiterhin eine Vorrichtung zur Entspannungsverdampfung. Der Ausdruck "Entspannungsverdampfung" bezeichnet im Rahmen der vorliegenden Anmeldung das Entstehen von Dampf bei Absenken des Druckes in einem mit Flüssigkeit befüllten, unter Überdruck (zur Umgebung) stehenden geschlossenen Raum. Ein entsprechender Überdruck lässt sich beispielsweise erzeugen, indem eine Menge der kosmetischen Zubereitung a) in einem abgeschlossenen Raum auf eine Temperatur T₁ erhitzt wird. In dem geschlossenen Raum hat die Flüssigkeit bei gegebener Temperatur T₁ einen Druck Sättigungsdruck p₁. Wird der geschlossenen Raum beispielsweise mittels eines Ventils zu einem nicht unter Überdruck stehenden Relaxationsraum mit dem Druck p₀ < p₁ geöffnet, so sinkt der Druck in dem zuvor geschlossenen Raum ab und im Rahmen der Ausbreitung des neuen Druckniveaus verdampft die kosmetische Zubereitung a), bzw. das in der kosmetischen Zubereitung enthaltene Lösungsmittel oder Teile dieses Lösungsmittels. Der entstehende Dampf oder Sprühnebel kann für die Applikation spezifischer kosmetischer Zubereitungen genutzt werden.

Wird die kosmetische Zubereitung a) also ausgehend von Standardbedingungen (T₀ = 25°C, p₀ = 1,000 bar) in einem geschlossenen Raum erhitzt, so resultiert neben einer erhöhten Temperatur weiterhin ein erhöhter Druck der kosmetischen Zubereitung a). Dieser erhöhte Druck kann in einem Relaxationsraum auf einen Druck p₀, zum Beispiel den umgebenden Luftdruck (p₀ = 1,000 bar), entlastet werden kann, wodurch mindestens teilweise eine Verdampfung der kosmetischen Zubereitung a) erreicht wird.

Die kosmetische Zubereitung a) kann unmittelbar in dem Raum entspannt werden, in welchem sie zuvor erhitzt wurde. Die erhitzte und unter Überdruck befindliche kosmetische Zubereitung a) kann alternativ aber auch nach dem Erhitzen in einen zweiten Raum transportiert werden, in welchem dann nachfolgend die Druckentlastung erfolgt.

Bei der Entspannungsverdampfung handelt es sich mit anderen Worten um ein Verfahren, bei welchem die kosmetische Zubereitung a) in einem geschlossenen Behälter mittels einer Heizvorrichtung auf Temperaturen oberhalb der Umgebungstemperatur erhitzt wird, wobei in dem Behälter ein Druck oberhalb des Umgebungsdrucks entsteht, und die erhitzte und unter erhöhtem Druck stehende kosmetische Zubereitung a) anschließend aus dem Behälter in die Umgebung entspannt wird.

Bei einer Vorrichtung zur Entspannungsverdampfung handelt es sich demnach um eine Vorrichtung, welche einen Behälter und eine Heizvorrichtung umfasst und derart ausgestaltet ist, dass eine kosmetische Zubereitung a) in dem geschlossenen Behälter mittels der Heizvorrichtung auf Temperaturen oberhalb der Umgebungstemperatur in einer Weise erhitzt werden kann, dass in dem Behälter ein Druck oberhalb des Umgebungsdrucks entsteht und die erhitzte und unter erhöhtem Druck stehende kosmetische Zubereitung a) aus dem Behälter in die Umgebung entspannt werden kann.

Gleichzeitig mit oder nach der Druckentlastung kann die kosmetische Zubereitung a) einer Düse zugeführt werden, mittels derer beispielsweise Eigenschaften des durch die Entspannungsverdampfung erzeugten Dampfes bzw. Sprühnebels, insbesondere die Tröpfchengröße oder die Tröpfchendichte aber auch die Sprühweite und die Form des Sprühkegels beeinflusst werden können. Der Einsatz von Düsen, vorzugsweise Zerstäuberdüsen, ist daher bevorzugt. Der spezifische Düsentyp oder die spezifische Düsengestaltung wird in Abhängigkeit von den jeweiligen Sprühnebeleigenschaften gezielt festgelegt.

Zusammenfassend verfügt eine bevorzugte Vorrichtung zur Entspannungsverdampfung über
b1) einen mittels eines Ventils zu verschließenden und zu öffnenden Behälter b1), welcher den geschlossenen Innenraum definiert, in dem die kosmetische Zubereitung aufgenommen werden kann,
b2) eine Heizvorrichtung b2), welche es ermöglicht eine in dem Behälter b1) befindliche kosmetische Zubereitung zu erhitzen.

Besonders bevorzugt ist der Einsatz einer zusätzlichen Düse b3), welche eine Zerstäubung der aus dem Behälter entweichenden kosmetischen Zubereitung a) ermöglicht. Alternativ zu einem Ventil kann auch ein vergleichbar wirkendes Schließelement, welches eine zugehörige Öffnung im Behälter durch entsprechende Positionsänderung verschließen oder freigeben kann, zum Einsatz kommen

Offenbart wird ein kosmetisches Produkt, umfassend
a) eine kosmetische Zubereitung, enthaltend bezogen auf ihr Gesamtgewicht
   a1) 45 bis 95 Gew.-% mindestens eines polaren Lösungsmittels;
   a2) 1,0 bis 25 Gew.-% mindestens eines Fettstoffs;
b) eine Vorrichtung zur Entspannungsverdampfung der kosmetischen Zubereitung a), wobei die Vorrichtung zur Entspannungsverdampfung einen Behälter b1) und eine Heizvorrichtung b2 umfasst und derart ausgestaltet ist, dass
   - die kosmetische Zubereitung a) in den Innenraum des Behälters b1) aufgenommen werden kann,
   - der mit der kosmetischen Zubereitung a) wenigstens anteilsweise befüllte Innenraum des Behälters b1) verschlossen werden kann,
   - die kosmetische Zubereitung a) in dem geschlossenen Innenraum des Behälters b1) mittels der Heizvorrichtung b2) unter Druckerhöhung erhitzt werden kann.

Offenbart wird ein kosmetisches Produkt, umfassend
a) eine kosmetische Zubereitung, enthaltend bezogen auf ihr Gesamtgewicht
   a1) 45 bis 95 Gew.-% mindestens eines polaren Lösungsmittels;
   a2) 1,0 bis 25 Gew.-% mindestens eines Fettstoffs;
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht ein in dem Behälter b1) befindliche kosmetische Zubereitung zu erhitzen
   b3) eine Düse b3), welche eine Zerstäubung der kosmetischen Zubereitung a) ermöglicht.

Offenbart wird ein kosmetisches Produkt, umfassend
a) eine kosmetische Zubereitung, enthaltend bezogen auf ihr Gesamtgewicht
   a1) 45 bis 95 Gew.-% mindestens eines polaren Lösungsmittels;
   a2) 1,0 bis 25 Gew.-% mindestens eines Fettstoffs;
b) eine Vorrichtung zur Entspannungsverdampfung der kosmetischen Zubereitung a), wobei die Vorrichtung zur Entspannungsverdampfung einen Behälter b1) und eine Heizvorrichtung b2 umfasst und derart ausgestaltet ist, dass
   - die kosmetische Zubereitung a) in den Innenraum des Behälters b1) aufgenommen werden kann,
   - der mit der kosmetischen Zubereitung a) wenigstens anteilsweise befüllte Innenraum des Behälters b1) verschlossen werden kann,
   - die kosmetische Zubereitung a) in dem geschlossenen Innenraum des Behälters b1) mittels der Heizvorrichtung b2) unter Druckerhöhung erhitzt werden kann,
   - die erhitzte kosmetische Zubereitung a) aus dem Innenraum des Behälters b1) unter Druckminderung in die Umgebung entspannt werden kann.

Der Behälter b1), in welchem die kosmetische Zubereitung erhitzt wird, ist in einer Weise ausgestaltet, die es ermöglicht, diesen Behälter während der Erhitzung der kosmetischen Zubereitung a) gegen die Umgebung vollständig abzuschließen und nach der Erhitzung, zur Ermöglichung Entspannungsverdampfung der kosmetischen Zubereitung a), zu öffnen. Dies kann beispielsweise durch ein Bauteil zur Durchflussregelung, insbesondere ein Ventil, gewährleistet werden.

Der Behälter b1), in welchem die kosmetische Zubereitung erhitzt wird, steht vorzugsweise in Kontakt mit einem weiteren Behälter, aus welchem die zur Entspannungsverdampfung vorgesehene Menge der kosmetischen Zubereitung vor der Erhitzung in den Behälter b1) überführt wird. Der Zugang zwischen diesem Vorratsbehälter und Behälter b1) ist dabei über eine entsprechende Vorrichtung, beispielsweise ein Ventil, zu öffnen und zu schließen. Dieser weitere Behälter ist vorzugsweise in Form eines Vorratsbehälters ausgestaltet, das heißt, er umfasst bevorzugt ein Vielfaches, beispielsweise mehr als das Zehnfache, vorzugsweise mehr als das Fünfzigfache, der für einen Verdampfungsvorgang notwendigen Menge der kosmetischen Zubereitung. Mit anderen Worten weist der weitere Behälter/Vorratsbehälter vorzugsweise ein Vielfaches, beispielsweise mehr als das Zehnfache Volumen, vorzugsweise mehr als das Zwanzigfache und insbesondere mehr als das Fünfzigfache Volumen des Behälters b1) auf.

Offenbart wird ein kosmetisches Produkt, umfassend
a) eine kosmetische Zubereitung, enthaltend bezogen auf ihr Gesamtgewicht
   a1) 45 bis 95 Gew.-% mindestens eines polaren Lösungsmittels;
   a2) 1,0 bis 25 Gew.-% mindestens eines Fettstoffs;
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht eine in dem geschlossenen Behälter b1) befindliche kosmetische Zubereitung zu erhitzen
   b3) eine Düse b3), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   o der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   o der Vorratsbehälter mindestens das Zehnfache Volumen, vorzugsweise mindestens das Zwanzigfache und insbesondere mindestens das Fünfzigfache Volumen des Behälters b1) aufweist.

Der Vorratsbehälter ist kein Druckbehälter und die in dem Vorratsbehälter befindliche kosmetische Zusammensetzung befindet sich nicht unter Druck, mit anderen Worten entspricht der Druck im Inneren des Vorratsbehälters dem Umgebungsdruck (auch Luftdruck oder Atmosphärendruck). So umfassen entsprechende kosmetische Produkte beispielsweise keine Treibmittel. Auch verfügt das kosmetische Produkt über keine Pumpvorrichtung, die geeignet ist, die kosmetische Zubereitung ohne die Einwirkung der Vorrichtung zur Entspannungsverdampfung in die Umgebung freizusetzen oder zu versprühen.

Offenbart wird ein kosmetisches Produkt, umfassend
a) eine kosmetische Zubereitung, enthaltend bezogen auf ihr Gesamtgewicht
   a1) 45 bis 95 Gew.-% mindestens eines polaren Lösungsmittels;
   a2) 1,0 bis 25 Gew.-% mindestens eines Fettstoffs;
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht eine in dem geschlossenen Behälter b1) befindliche kosmetische Zubereitung zu erhitzen
   b3) eine Düse b3), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   o der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   o der Vorratsbehälter mindestens das Zehnfache Volumen, vorzugsweise mindestens das Fünfzigfache Volumen des Behälters b1) aufweist;
   o der Druck im Inneren des Vorratsbehälters dem Umgebungsdruck entspricht.

Offenbart wird ein kosmetisches Produkt, umfassend
a) eine kosmetische Zubereitung, enthaltend bezogen auf ihr Gesamtgewicht
   a1) 45 bis 95 Gew.-% mindestens eines polaren Lösungsmittels;
   a2) 1,0 bis 25 Gew.-% mindestens eines Fettstoffs;
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht eine in dem geschlossenen Behälter b1) befindliche kosmetische Zubereitung zu erhitzen
   b3) eine Düse b3), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   o der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   ∘ der Vorratsbehälter mindestens das Zehnfache Volumen, vorzugsweise mindestens das Fünfzigfache Volumen des Behälters b1) aufweist;
   o der Druck im Inneren des Vorratsbehälters dem Umgebungsdruck entspricht und das kosmetische Produkt kein Treibmittel umfasst.

Bevorzugt werden weiterhin kosmetische Produkte, umfassend
a) eine kosmetische Zubereitung, enthaltend bezogen auf ihr Gesamtgewicht
   a1) 45 bis 95 Gew.-% mindestens eines polaren Lösungsmittels;
   a2) 1,0 bis 25 Gew.-% mindestens eines Fettstoffs;
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht eine in dem geschlossenen Behälter b1) befindliche kosmetische Zubereitung zu erhitzen
   b3) eine Düse b3), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   o der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   ∘ der Vorratsbehälter mindestens das Zehnfache Volumen, vorzugsweise mindestens das Fünfzigfache Volumen des Behälters b1) aufweist;
   o der Druck im Inneren des Vorratsbehälters dem Umgebungsdruck entspricht,
wobei das kosmetische Produkt keine Pumpvorrichtung aufweist, die geeignet ist, die kosmetische Zubereitung a) ohne die Einwirkung der Vorrichtung zur Entspannungsverdampfung freizusetzen oder zu versprühen.

Zusammenfassend ist ein besonders bevorzugter Gegenstand der vorliegenden Erfindung daher ein kosmetisches Produkt, umfassend
a) eine kosmetische Zubereitung, enthaltend bezogen auf ihr Gesamtgewicht
   a1) 45 bis 95 Gew.-% mindestens eines polaren Lösungsmittels;
   a2) 1,0 bis 25 Gew.-% mindestens eines Fettstoffs;
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht eine in dem geschlossenen Behälter b1) befindliche kosmetische Zubereitung zu erhitzen
   b3) eine Düse b3), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   o der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   ∘ der Vorratsbehälter mindestens das Zehnfache Volumen, vorzugsweise mindestens das Fünfzigfache Volumen des Behälters b1) aufweist;
   ∘ der Druck im Inneren des Vorratsbehälters dem Umgebungsdruck entspricht und das kosmetische Produkt kein Treibmittel umfasst,
wobei das kosmetische Produkt keine Pumpvorrichtung aufweist, die geeignet ist, die kosmetische Zubereitung a) ohne die Einwirkung der Vorrichtung zur Entspannungsverdampfung freizusetzen oder zu versprühen.

Neben den beiden zuvor beschriebenen Bestandteilen a1) und a2) können die erfindungsgemäßen kosmetischen Zubereitungen a) weitere Wirk- oder Hilfsstoffe enthalten, wobei insbesondere solche Wirk- oder Hilfsstoffe bevorzugt werden, welche die Herstellbarkeit, Applizierbarkeit und/oder kosmetische Wirkung erfindungsgemäßer kosmetischer Zubereitungen verbessern.

Ein erstes Beispiel für einen bevorzugten weiteren Wirk- und Hilfsstoff sind die filmbildenden Polymere a3), deren Einsatz in den erfindungsgemäßen kosmetischen Zubereitungen a) besonders bevorzugt ist.

Als filmbildende Polymere a3) eignen sich permanent als auch temporär kationische, anionische, nichtionische oder amphotere Polymere. Diese filmbildenden Polymere können synthetischen oder natürlichen Ursprungs sein. Bevorzugte kosmetische Zubereitung a) enthalten bezogen auf ihr Gesamtgewicht 0,05 bis 20 Gew.-%, vorzugsweise 0,05 bis 15 Gew.-%, und insbesondere 0,1 bis 10 Gew.-% eines filmbildenden Polymers a3). Dieses Polymer a4) ist von dem Polymer a2) verschieden.

Beispiele für gebräuchliche filmbildende Polymere a3) sind Acrylamide/Ammonium Acrylate Copolymer, Acrylamides/DMAPA Acrylates/Methoxy PEG Methacrylate Copolymer, Acrylamidopropyltrimonium Chloride/Acrylamide Copolymer, Acrylamidopropyltrimonium Chloride/Acrylates Copolymer, Acrylates/Acetoacetoxyethyl Methacrylate Copolymer, Acrylates/Acrylamide Copolymer, Acrylates/Ammonium Methacrylate Copolymer, Acrylates/t-Butylacrylamide Copolymer, Acrylates Copolymer, Acrylates/C1-2 Succinates/Hydroxyacrylates Copolymer, Acrylates/Lauryl Acrylate/Stearyl Acrylate/Ethylamine Oxide Methacrylate Copolymer, Acrylates/Octylacrylamide Copolymer, Acrylates/Octylacrylamide/Diphenyl Amodimethicone Copolymer, Acrylates/Stearyl Acrylate/Ethylamine Oxide Methacrylate Copolymer, Acrylates/VA Copolymer, Acrylates/VP Copolymer, Adipic Acid/Diethylenetriamine Copolymer, Adipic Acid/Dimethylaminohydroxypropyl Diethylenetriamine Copolymer, Adipic Acid/Epoxypropyl Diethylenetriamine Copolymer, Adipic Acid/Isophthalic Acid/Neopentyl Glycol/Trimethylolpropane Copolymer, Allyl Stearate/VA Copolymer, Aminoethylacrylate Phosphate/Acrylates Copolymer, Aminoethylpropanediol-Acrylates/Acrylamide Copolymer, Aminoethylpropanediol-AMPD-Acrylates/Diacetoneacrylamide Copolymer, Ammonium VA/Acrylates Copolymer, AMPD-Acrylates/Diacetoneacrylamide Copolymer, AMP-Acrylates/Allyl Methacrylate Copolymer, AMP-Acrylates/C1-18 Alkyl Acrylates/C1-8 Alkyl Acrylamide Copolymer, AMP-Acrylates/Diacetoneacrylamide Copolymer, AMP-Acrylates/Dimethylaminoethylmethacrylate Copolymer, Bacillus/Rice Bran Extract/Soybean Extract Ferment Filtrate, Bis-Butyloxyamodimethicone/PEG-60 Copolymer, Butyl Acrylate/Ethylhexyl Methacrylate Copolymer, Butyl Acrylate/Hydroxypropyl Dimethicone Acrylate Copolymer, Butylated PVP, Butyl Ester of Ethylene/MA Copolymer, Butyl Ester of PVM/MA Copolymer, Calcium/Sodium PVM/MA Copolymer, Corn Starch/Acrylamide/ Sodium Acrylate Copolymer, Diethylene Glycolamine/Epichlorohydrin/Piperazine Copolymer, Dimethicone Crosspolymer, Diphenyl Amodimethicone, Ethyl Ester of PVM/MA Copolymer, Hydrolyzed Wheat Protein/PVP Crosspolymer, Isobutylene/Ethylmaleimide/ Hydroxyethylmaleimide Copolymer, Isobutylene/MA Copolymer, Isobutylmethacrylate/Bis-Hydroxypropyl Dimethicone Acrylate Copolymer, Isopropyl Ester of PVM/MA Copolymer, Lauryl Acrylate Crosspolymer, Lauryl Methacrylate/Glycol Dimethacrylate Crosspolymer, MEA-Sulfite, Methacrylic Acid/Sodium Acrylamidomethyl Propane Sulfonate Copolymer, Methacryloyl Ethyl Betaine/Acrylates Copolymer, Octylacrylamide/Acrylates/Butylaminoethyl Methacrylate Copolymer, PEG/PPG-25/25 Dimethicone/Acrylates Copolymer, PEG-8/SMDI Copolymer, Polyacrylamide, Polyacrylate-6, Polybeta-Alanine/Glutaric Acid Crosspolymer, Polybutylene Terephthalate, Polyester-1, Polyethylacrylate, Polyethylene Terephthalate, Polymethacryloyl Ethyl Betaine, Polypentaerythrityl Terephthalate, Polyperfluoroperhydrophenanthrene, Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-39, Polyquaternium-45, Polyquaternium-46, Polyquaternium-47, Polyquaternium-48, Polyquaternium-49, Polyquaternium-50, Polyquaternium-55, Polyquaternium-56, Polysilicone-9, Polyurethane-1, Polyurethane-6, Polyurethane-10, Polyvinyl Acetate, Polyvinyl Butyral, Polyvinylcaprolactam, Polyvinylformamide, Polyvinyl Imidazolinium Acetate, Polyvinyl Methyl Ether, Potassium Butyl Ester of PVM/MA Copolymer, Potassium Ethyl Ester of PVM/MA Copolymer, PPG-70 Polyglyceryl-10 Ether, PPG-12/SMDI Copolymer, PPG-51/SMDI Copolymer, PPG-10 Sorbitol, PVM/MA Copolymer, PVP, PVP/VA/Itaconic Acid Copolymer, PVP/VA/Vinyl Propionate Copolymer, Rhizobian Gum, Rosin Acrylate, Shellac, Sodium Butyl Ester of PVM/MA Copolymer, Sodium Ethyl Ester of PVM/MA Copolymer, Sodium Polyacrylate, Sterculia Urens Gum, Terephthalic Acid/Isophthalic Acid/Sodium Isophthalic Acid Sulfonate/Glycol Copolymer, Trimethylolpropane Triacrylate, Trimethylsiloxysilylcarbamoyl Pullulan, VA/Crotonates Copolymer, VA/Crotonates/Methacryloxybenzophenone-1 Copolymer, VA/Crotonates/Vinyl Neodecanoate Copolymer, VA/Crotonates/Vinyl Propionate Copolymer, VA/DBM Copolymer, VA/Vinyl Butyl Benzoate/Crotonates Copolymer, Vinylamine/Vinyl Alcohol Copolymer, Vinyl Caprolactam/VP/Dimethylaminoethyl Methacrylate Copolymer, VP/Acrylates/Lauryl Methacrylate Copolymer, VP/Dimethylaminoethylmethacrylate Copolymer, VP/DMAPA Acrylates Copolymer, VP/Hexadecene Copolymer, VP/VA Copolymer, VP/Vinyl Caprolactam/DMAPA Acrylates Copolymer, Yeast Palmitate und Styrene/VP Copolymer.

Eine erste Gruppe besonders bevorzugter Polymere a3) bilden die Vinylpyrrolidon Homo- oder Copolymere. Mit besonderem Vorzug eingesetzte Polymere sind:
- Polyvinylpyrrolidone (INCI-Bezeichnung PVP; CAS-Nummer 9003-39-8), wie sie beispielsweise unter der Bezeichnung Luviskol® (BASF) vertrieben werden;
- Copolymere von Vinylpyrrolidon und Vinylacetat (INCI-Bezeichnung VP/VA Copolymer; CAS-Nummer: 25086-89-9), wie sie beispielsweise unter der Bezeichnung Luviskol® VA (BASF) vertrieben werden;
- Copolymere von Vinylpyrrolidon und Dimethylaminopropylmethacrylamid (INCI-Bezeichnung: VP/DMAPA Acrylates Copolymer; CAS-Nummer: 175893-71-7), wie sie beispielsweise unter der Bezeichnung Styleze® CC-10 (Ashland) vertrieben werden;
- Copolymere von Vinylpyrrolidon mit Vinylcaprolactam und Dimethylaminoethylmethacrylat (INCI-Bezeichnung: Vinyl Caprolactam/VP/Dimethylaminoethyl Methacrylate Copolymer; CAS-Nummer: 102972-64-5), wie sie beispielsweise unter der Bezeichnung Advantage® LC (Ashland) vertrieben werden.

Aufgrund ihrer kosmetischen Wirkung in Kombination mit den Fettstoffen a2) erfindungsgemäß bevorzugt eingesetzte filmbildenden Polymere sind insbesondere ausgewählt aus der Gruppe der Vinylpyrrolidion Homo- und Copolymere, vorzugsweise aus der Gruppe der Polyvinylpyrrolidone, der Copolymere von Vinylpyrrolidon und Vinylacetat, der Copolymere von Vinylpyrrolidon und Dimethylaminopropylmethacrylamid sowie der Copolymere von Vinylpyrrolidon mit Vinylcaprolactam und Dimethylaminoethylmethacrylat.

Eine zweite Gruppe besonders bevorzugter Polymere a3) bilden die Copolymere von i) N-tert-Octylacrylamid, ii) Acrylsäure, iii) tert.-Butylaminoethylmethacrylat iv) sowie gegebenenfalls weiteren Monomeren.

Bevorzugte Copolymere a2) bestehen bevorzugt zu mindestens 90 Gew.-%, vorzugsweise zu mindestens 95 Gew.-% und insbesondere zu mindestens 97 Gew.-% aus den Monomeren N-tert-Octylacrylamid, Acrylsäure und tert.-Butylaminoethylmethacrylat. Besonders bevorzugte Copolymere a2) wurden ausschließlich aus den Monomeren N-tert-Octylacrylamid, Acrylsäure und tert.-Butylaminoethylmethacrylat erhalten.

Besonders bevorzugt sind Copolymere a2) aus den Monomeren i) N-tert-Octylacrylamid, ii) Acrylsäure, iii) tert.-Butylaminoethylmethacrylat, iv) Methylmethacrylat und v) Hydroxypropylmethacrylat.

Die zuvor beschriebenen Copolymere a2) werden beispielsweise unter der Bezeichnung Amphomer® (INCI-Bezeichnung: Octylacrylamide / Acrylates / Butylaminoethyl Methacrylate Copolymer; CAS Nummer 70801-07-9) von der Firma National Starch vertrieben.

Ein zweites Beispiel für einen bevorzugten weiteren Wirk- und Hilfsstoff sind die Verdicker a4). Bevorzugte Verdicker sind ausgewählt aus der Gruppe der polymeren organischen Verdicker. Die polymeren organischen Verdicker können vernetzt oder unvernetzt sein.

In Bezug auf die Herstellbarkeit, Applizierbarkeit und kosmetische Wirkung erfindungsgemäßer kosmetischer Zusammensetzungen hat es sich als vorteilhaft erwiesen, wenn der Gewichtsanteil des Verdickers a4) am Gesamtgewicht der kosmetischen Zubereitung a) 0,05 bis 10 Gew.-%, vorzugsweise 0,05 bis 7,0 und insbesondere 0,1 bis 5,0 Gew.-% beträgt.

Beispiele für gebräuchliche Verdicker a4) sind polymere Verdickungsmittel mit den INCI-Bezeichnungen Acrylamides Copolymer, Acrylamide/Sodium Acrylate Copolymer, Acrylamide/Sodium Acryloyldimethyltaurate Copolymer, Acrylates/Acetoacetoxyethyl Methacrylate Copolymer, Acrylic Acid/Acrylonitrogens Copolymer, Agar, Agarose, Alcaligenes Polysaccharides, Algin, Alginic Acid, Ammonium Acrylates/Acrylonitrogens Copolymer, Ammonium Acrylates Copolymer, Ammonium Acryloyldimethyltaurate/Vinyl Formamide Copolymer, Ammonium Acryloyldimethyltaurate/VP Copolymer, Ammonium Alginate, Ammonium Polyacryloyldimethyl Taurate, Amylopectin, Ascorbyl Methylsilanol Pectinate, Astragalus Gummifer Gum, Attapulgite, Avena Sativa (Oat) Kernel Flour, Bentonite, Butoxy Chitosan, Caesalpinia Spinosa Gum, Calcium Alginate, Calcium Carboxymethyl Cellulose, Calcium Carrageenan, Calcium Potassium Carbomer, Calcium Starch Octenylsuccinate, C20-40 Alkyl Stearate, Carboxybutyl Chitosan, Carboxymethyl Chitin, Carboxymethyl Chitosan, Carboxymethyl Dextran, Carboxymethyl Hydroxyethylcellulose, Carboxymethyl Hydroxypropyl Guar, Cellulose Acetate Propionate Carboxylate, Cellulose Gum, Ceratonia Siliqua Gum, Cetyl Hydroxyethylcellulose, Cholesterol/HDI/Pullulan Copolymer, Cholesteryl Hexyl Dicarbamate Pullulan, Cyamopsis Tetragonoloba (Guar) Gum, Diglycol/CHDM/Isophthalates/SIP Copolymer, Dihydrogenated Tallow Benzylmonium Hectorite, Dimethicone Crosspolymer-2, Dimethicone Propyl PG-Betaine, DMAPA Acrylates/Acrylic Acid/Acrylonitrogens Copolymer, Ethylene/Sodium Acrylate Copolymer, Gelatin, Gellan Gum, Glyceryl Alginate, Glycine Soja (Soybean) Flour, Guar Hydroxypropyltrimonium Chloride, Hectorite, Hydrated Silica, Hydrogenated Potato Starch, Hydroxybutyl Methylcellulose, Hydroxyethyl Acrylate/Sodium Acryloyldimethyl Taurate Copolymer, Hydroxyethylcellulose, Hydroxyethyl Chitosan, Hydroxyethyl Ethylcellulose, Hydroxypropylcellulose, Hydroxypropyl Chitosan, Hydroxypropyl Ethylenediamine Carbomer, Hydroxypropyl Guar, Hydroxypropyl Methylcellulose, Hydroxypropyl Methylcellulose Stearoxy Ether, Hydroxystearamide MEA, Isobutylene/Sodium Maleate Copolymer, Lithium Magnesium Silicate, Lithium Magnesium Sodium Silicate, Macrocystis Pyrifera (Kelp), Magnesium Alginate, Magnesium Aluminum Silicate, Magnesium Silicate, Magnesium Trisilicate, Methoxy PEG-22/Dodecyl Glycol Copolymer, Methylcellulose, Methyl Ethylcellulose, Methyl Hydroxyethylcellulose, Microcrystalline Cellulose, Montmorillonite, Moroccan Lava Clay, Natto Gum, Nonoxynyl Hydroxyethylcellulose, Octadecene/MA Copolymer, Pectin, PEG-800, PEG-Crosspolymer, PEG-150/Decyl Alcohol/SMDI Copolymer, PEG-175 Diisostearate, PEG-190 Distearate, PEG-15 Glyceryl Tristearate, PEG-140 Glyceryl Tristearate, PEG-240/HDI Copolymer Bis-Decyltetradeceth-20 Ether, PEG-100/IPDI Copolymer, PEG-180/Laureth-50/TMMG Copolymer, PEG-10/Lauryl Dimethicone Crosspolymer, PEG-15/Lauryl Dimethicone Crosspolymer, PEG-2M, PEG-5M, PEG-7M, PEG-9M, PEG-14M, PEG-20M, PEG-23M, PEG-25M, PEG-45M, PEG-65M, PEG-90M, PEG-115M, PEG-160M, PEG-120 Methyl Glucose Trioleate, PEG-180/Octoxynol-40/TMMG Copolymer, PEG-150 Pentaerythrityl Tetrastearate, PEG-4 Rapeseedamide, PEG-150/Stearyl Alcohol/SMDI Copolymer, Polyacrylate-3, Polyacrylic Acid, Polycyclopentadiene, Polyether-1, Polyethylene/Isopropyl Maleate/MA Copolyol, Polymethacrylic Acid, Polyquaternium-52, Polyvinyl Alcohol, Potassium Alginate, Potassium Aluminum Polyacrylate, Potassium Carbomer, Potassium Carrageenan, Potassium Polyacrylate, Potato Starch Modified, PPG-14 Laureth-60 Hexyl Dicarbamate, PPG-14 Laureth-60 Isophoryl Dicarbamate, PPG-14 Palmeth-60 Hexyl Dicarbamate, Propylene Glycol Alginate, PVP/Decene Copolymer, PVP Montmorillonite, Rhizobian Gum, Ricinoleic Acid/Adipic Acid/AEEA Copolymer, Sclerotium Gum, Sodium Acrylate/Acryloyldimethyl Taurate Copolymer, Sodium Acrylates/Acrolein Copolymer, Sodium Acrylates/Acrylonitrogens Copolymer, Sodium Acrylates Copolymer, Sodium Acrylates/Vinyl Isodecanoate Crosspolymer, Sodium Acrylate/Vinyl Alcohol Copolymer, Sodium Carbomer, Sodium Carboxymethyl Chitin, Sodium Carboxymethyl Dextran, Sodium Carboxymethyl Beta-Glucan, Sodium Carboxymethyl Starch, Sodium Carrageenan, Sodium Cellulose Sulfate, Sodium Cyclodextrin Sulfate, Sodium Hydroxypropyl Starch Phosphate, Sodium Isooctylene/MA Copolymer, Sodium Magnesium Fluorosilicate, Sodium Polyacrylate, Sodium Polyacrylate Starch, Sodium Polyacryloyldimethyl Taurate, Sodium Polymethacrylate, Sodium Polystyrene Sulfonate, Sodium Silicoaluminate, Sodium Starch Octenylsuccinate, Sodium Stearoxy PG-Hydroxyethylcellulose Sulfonate, Sodium Styrene/Acrylates Copolymer, Sodium Tauride Acrylates/Acrylic Acid/Acrylonitrogens Copolymer, Solanum Tuberosum (Potato) Starch, Starch/Acrylates/Acrylamide Copolymer, Starch Hydroxypropyltrimonium Chloride, Steareth-60 Cetyl Ether, Steareth-100/PEG-136/HDI Copolymer, Sterculia Urens Gum, Synthetic Fluorphlogopite, Tamarindus Indica Seed Gum, Tapioca Starch, TEA-Alginate, TEA-Carbomer, Triticum Vulgare (Wheat) Starch, Tromethamine Acrylates/Acrylonitrogens Copolymer, Tromethamine Magnesium Aluminum Silicate, Welan Gum, Yeast Beta-Glucan, Yeast Polysaccharides, Zea Mays (Corn) Starch.

Eine erste Gruppe besonders bevorzugter Verdicker a4) enthalten mindestens eine Struktureinheit ausgewählt aus mindestens einer Struktureinheit der Formel (I) oder deren Salzformen oder mindestens einer Struktureinheit (II) oder deren Salzformen, 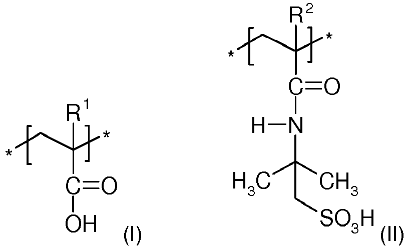 worin R¹ und R² unabhängig voneinander für ein Wasserstoffatom oder eine Methylgruppe stehen.

Gemäß obiger Formeln und allen folgenden Formeln steht eine chemische Bindung, die mit dem Symbol * gekennzeichnet ist, für eine freie Valenz des entsprechenden Strukturfragments.

Besonders bevorzugte anionische, verdickend wirkende Polymere enthalten mindestens eine Struktureinheit der Formel (I). Acrylsäurehomopolymere bilden eine erste Gruppe besonders bevorzugter Verdicker a4).

Besonders bevorzugte Verdicker sind
- Polyacrylsäuren mit der INCI-Bezeichnung Carbomer, wie sie beispielsweise von der Firma 3V Sigma unter dem Handelsnamen Synthalen® K oder von der Firma Lubrizol unter dem Handelsnamen Carbopol vertrieben werden.

Weitere besonders bevorzugte anionische, verdickend wirkende Polymere enthalten mindestens eine Struktureinheit der Formel (II). Sulfonsäurecopolymere bilden eine zweite Gruppe besonders bevorzugter Verdicker a4). Bevorzugt werden insbesondere die Copolymere von Sulfsonsäurehaltigen Monomeren mit Acrylsäure.

Besonders bevorzugte Verdicker sind weiterhin
- Sulfonsäurecopolymere mit der INCI-Bezeichnung Sodium Acrylate/Sodium Acryloyldimethyl Taurate Copolymer, wie sie beispielsweise von der Firma Seppic unter dem Handelsnamen Simulgel® EG vertrieben werden;
- Sulfonsäurecopolymere mit der INCI-Bezeichnung Sodium Acrylate/Acryloyldimethyl Taurate/Dimethylacrylamide Crosspolymer, wie sie beispielsweise von der Firma Seppic unter dem Handelsnamen Simulgel® SMS 88 vertrieben werden.

Eine zweite besonders bevorzugte Gruppe von Verdickern a4) bilden die polymeren, anionischen, amphiphilen Verdicker. Entsprechende Verdicker umfassen bevorzugt mindestens eine Struktureinheit der Formel (III) und mindestens eine Struktureinheit der Formel (IV), 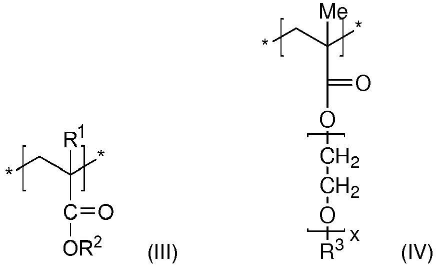 worin
R¹ steht für ein Wasserstoffatom oder eine Methylgruppe,
R² steht für ein Wasserstoffatom oder eine (C₁ bis C₆)-Alkylgruppe
R³ steht für eine (C₈ bis C₃₀)-Alkylgruppe,
M⁺ steht für ein physiologisch verträgliches Kation und
x steht für eine ganze Zahl von 0 bis 35.

Bevorzugt sind dabei insbesondere die Verdicker mit den INCI-Bezeichnungen Acrylates/Beheneth-25 Methacrylate Copolymer, Acrylates/Ceteth-20 Itaconate Copolymer, Acrylates/Ceteth-20 Methacrylate Copolymer, Acrylates/Laureth-25 Methacrylate Copolymer, Acrylates/Palmeth-20 Acrylate Copolymer, Acrylates/Palmeth-25 Acrylate Copolymer, Acrylates/Palmeth-25 Itaconate Copolymer und Acrylates/Steareth-50 Acrylate Copolymer.

Besonders bevorzugte Verdicker sind
- Verdicker mit der INCI-Bezeichnung Acrylates/Steareth-20 Methacrylate Copolymer, wie sie beispielsweise unter der Handelsnamen Aculyn® 22 von der Firma Rohm&Haas vertrieben werden;
- Verdicker mit der INCI-Bezeichnung Acrylates/Steareth-20 Methacrylate Crosspolymer, wie sie beispielsweise unter der Handelsnamen Aculyn® 88 von der Firma Rohm&Haas vertrieben werden;
- Verdicker mit der INCI-Bezeichnung Acrylates/Steareth-20 Itaconate Copolymer, wie sie beispielsweise unter dem Handelsnamen Structure 2001 von der Firma National Starch vertrieben werden.

Weitere polymere, anionische, amphiphile Verdicker zeichnen sich durch langkettige Alkylsubstituenten aus. Zu dieser Gruppe zählen beispielsweise die Verbindungen mit den INCI-Bezeichnungen Acrylates/Stearyl Methacrylate Copolymer, Acrylates/Vinyl Isodecanoate Crosspolymer.

Eine letzte Gruppe besonders bevorzugter Verdicker sind schließlich
- Verdicker mit der INCI-Bezeichnung Acrylates/C₁₀₋₃₀ Alkyl Acrylate Crosspolymer, wie sie beispielsweise unter dem Handelsnamen Carbopol Ultrez 21 von der Firma Lubrizol vertreiben werden.

Ein drittes Beispiel für einen bevorzugten weiteren Wirk- und Hilfsstoff sind die Emulgatoren a5). In Bezug auf die Herstellbarkeit, Applizierbarkeit und kosmetische Wirkung erfindungsgemäßer kosmetischer Zusammensetzungen hat es sich als vorteilhaft erwiesen, wenn der Gewichtsanteil des Emulgators a5) am Gesamtgewicht der kosmetischen Zubereitung a) 0,02 bis 4,0 Gew.-%, vorzugsweise 0,05 bis 2,0 Gew.-% und insbesondere 0,1 bis 1,0 Gew.-% beträgt.

Eine erste Gruppe bevorzugter Emulgatoren bilden die Poly-(C₂-C₃)alkylenglycol-modifizierten Silicone a5). Besonders bevorzugt sind Poly-(C₂-C₃)alkylenglycol-modifizierten Silicone aus der Gruppe der alkoxylierten Dimethicone, insbesondere der
- ethoxylierten Dimethicone mit der INCI-Bezeichnung PEG-x Dimethicone mit x = 2 bis 20, vorzugsweise 3 bis 17 und insbesondere 11 oder 12;
- der ethoxylierten Dimethicone mit der INCI-Bezeichnung Bis-PEG-y Dimethicone mit x= 3 bis 25, vorzugsweise 4 bis 20;
- der ethoxylierten/propoxylierten Dimethicone mit der INCI-Bezeichnung PEG/PPG a/b Dimethicone, wobei a und b unabhängig voneinander für Zahlen von 2 bis 30, vorzugsweise von 12 bis 24 und insbesondere von 14 bis 20 stehen;
- der ethoxylierten/propoxylierten Dimethicone mit der INCI-Bezeichnung Bis-PEG/PPG-c/d Dimethicone, wobei c und d unabhängig voneinander für Zahlen von 10 bis 25, bevorzugt 14 bis 20 und insbesondere von 14 bis 16 stehen
- der ethoxylierten/propoxylierten Dimethicone mit der INCI-Bezeichnung Bis-PEG/PPG-e/f PEG/PPG g/h Dimethicone, wobei e, f, g und h unabhängig voneinander für Zahlen von 10 bis 20, vorzugsweise 14 bis 18 und insbesondere 16 stehen.

Besonders bevorzugt ist der Einsatz von Poly-(C₂-C₃)alkylenglycol-modifizierten Silicon a5) aus der Gruppe der ethoxylierten/propoxylierten Dimethicone.

Eine zweite Gruppe bevorzugter Emulgatoren bilden die ethoxylierten Fettalkohole. Unter einem ethoxylierten Fettalkohol wird im Sinne der Erfindung ein Anlagerungsprodukt von Ethylenoxid an einen Fettalkohol verstanden, wobei der Ethoxylierungsgrad die Molmenge Ethylenoxid (EO) angibt, die durchschnittlich pro Mol Fettalkohol angelagert wurde. Polyethoxylierte Fettalkohole gemäß vorliegender Erfindung sind bevorzugt ausgewählt aus polyethoxylierten, linearen oder verzweigten, gesättigten oder ungesättigten Fettalkoholen, bevorzugt einer Kettenlänge mit 8 bis 22 Kohlenstoffatomen.

Je nach Herstellungsmethode fallen die erfindungsgemäßen ethoxylierten Fettalkohole als ein Gemisch mit einer unterschiedlichen Ethoxylierungsgradverteilung an. Im Sinne der Erfindung werden diese Tenside daher nach dem durchschnittlichen Ethoxylierungsgrad gekennzeichnet. Dieser ist üblicherweise als Ziffer hinter dem Fettalkohol-Suffix "eth-" in der INCI-Bezeichnung erkennbar. Bevorzugt enthalten die erfindungsgemäßen Mittel polyethoxylierte Fettalkohole, die einen Ethoxylierungsgrad von 2 bis 29, bevorzugt mit einem Ethoxylierungsgrad von 2 bis 25, weiter bevorzugt von 3 bis 20 aufweisen.

Bevorzugte ethoxylierte Fettalkohole mit einem durchschnittlichen Ethoxylierungsgrad von 2 bis 29 sind beispielsweise Laureth-2, Oleth-2, Ceteareth-2, Laneth-2, Laureth-3, Oleth-3, Ceteareth-3, Laureth-4, Oleth-4, Ceteareth-4, Laneth-4, Laureth-5, Oleth-5, Ceteareth-5, Laneth-5, Deceth-7, Laureth-7, Oleth-7, Coceth-7, Ceteth-7, Ceteareth-7, C11-15 Pareth-7, Laureth-9, Oleth-9, Ceteareth-9, Laureth-10, Oleth-10, Beheneth-10, Ceteareth-10, Laureth-12, Ceteareth-12, Trideceth-12, Ceteth-15, Laneth-15, Ceteareth-15, Laneth-16, Ceteth-16, Oleth-16, Steareth-16, Oleth-20, Ceteth-20, Ceteareth-20, Laneth-20, Steareth-21, Ceteareth-23, Ceteareth-25, Ceteareth-27. Besonders bevorzugt ist der Einsatz eines Gemisches von Steareth-2 und Steareth-21.

Als weitere geeignete Wirk- oder Hilfsstoffe sind insbesondere zusätzliche Pflegestoffe zu nennen.

Als Pflegestoff kann das Mittel beispielsweise mindestens ein Proteinhydrolysat und/oder eines seiner Derivate enthalten. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Als Pflegestoff kann das erfindungsgemäße Mittel weiterhin mindestens ein Vitamin, ein Provitamin, eine Vitaminvorstufe und/oder eines derer Derivate enthalten. Dabei sind erfindungsgemäß solche Vitamine, Provitamine und Vitaminvorstufen bevorzugt, die üblicherweise den Gruppen A, B, C, E, F und H zugeordnet werden.

Weitere Pflegestoffe sind Panthenol, Coffein, Nicotinamid und Sorbitol.

Als Pflegestoff können die erfindungsgemäßen Mittel weiterhin mindestens einen Pflanzenextrakt, aber auch Mono- bzw. Oligosaccharide und/oder Lipide enthalten.

Die Zusammensetzung einiger besonders bevorzugter erfindungsgemäßer kosmetischer Zubereitungen kann den folgenden Tabellen entnommen werden (Angaben in Gew.-% bezogen auf das Gesamtgewicht des kosmetischen Mittels sofern nicht anders angegeben). Bezüglich weiterer bevorzugter Ausführungsformen dieser besonders bevorzugten Zusammensetzungen gilt mutatis mutandis das zuvor zu den erfindungsgemäßen kosmetischen Zubereitungen a) Gesagte.

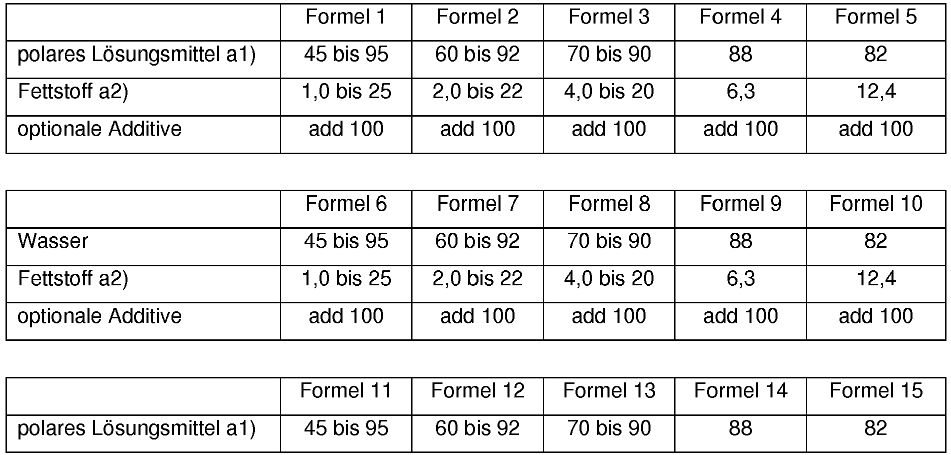

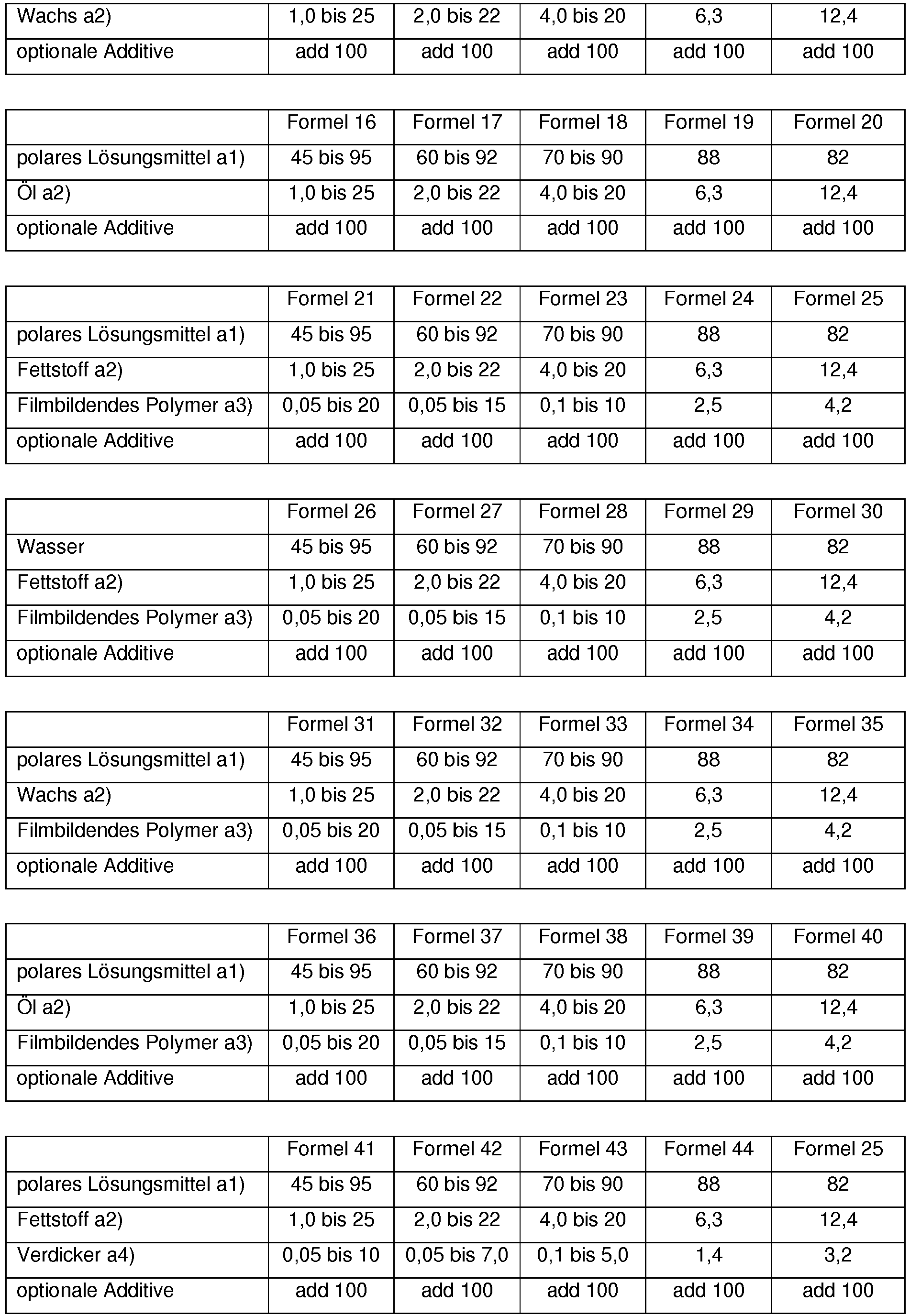

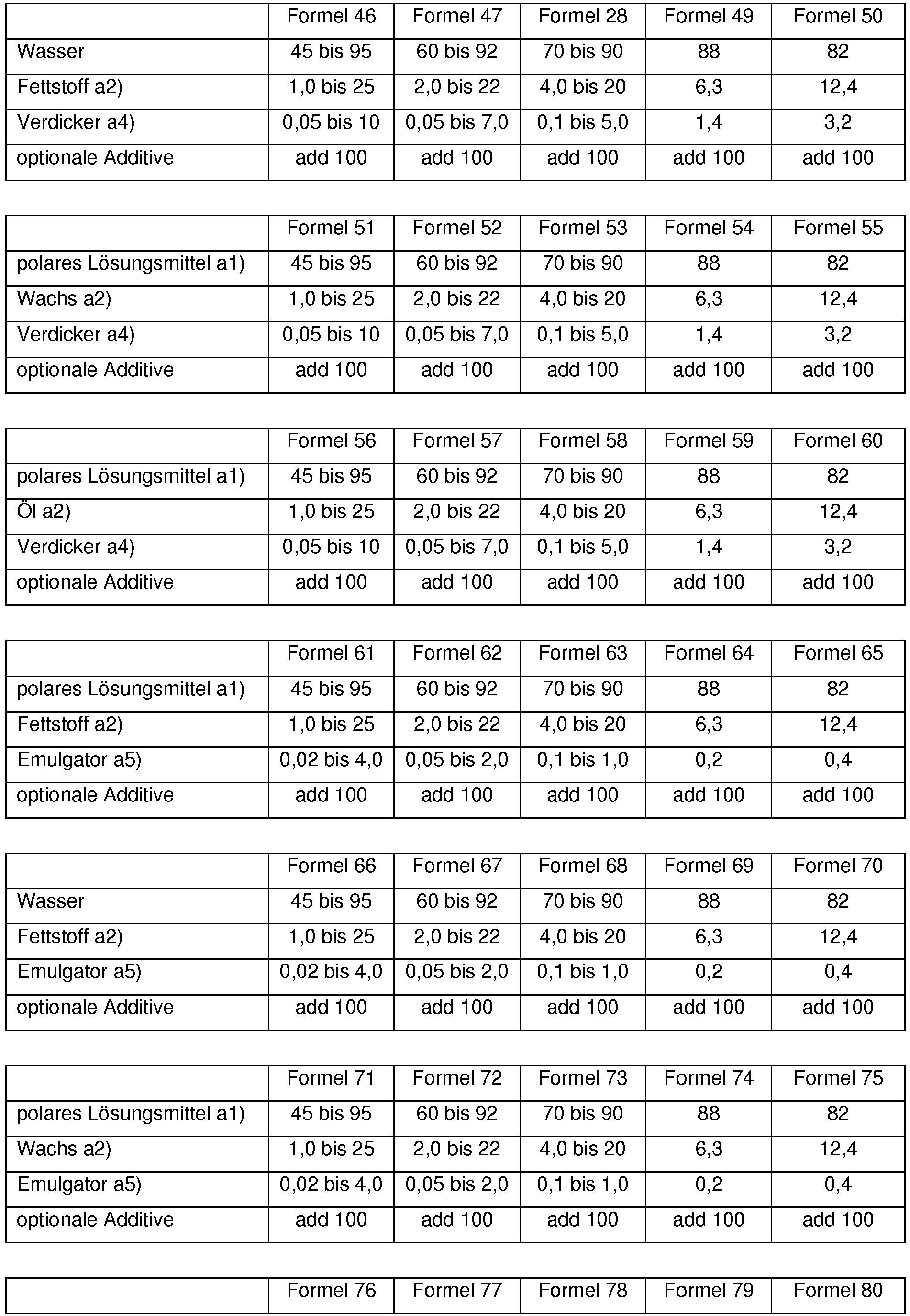

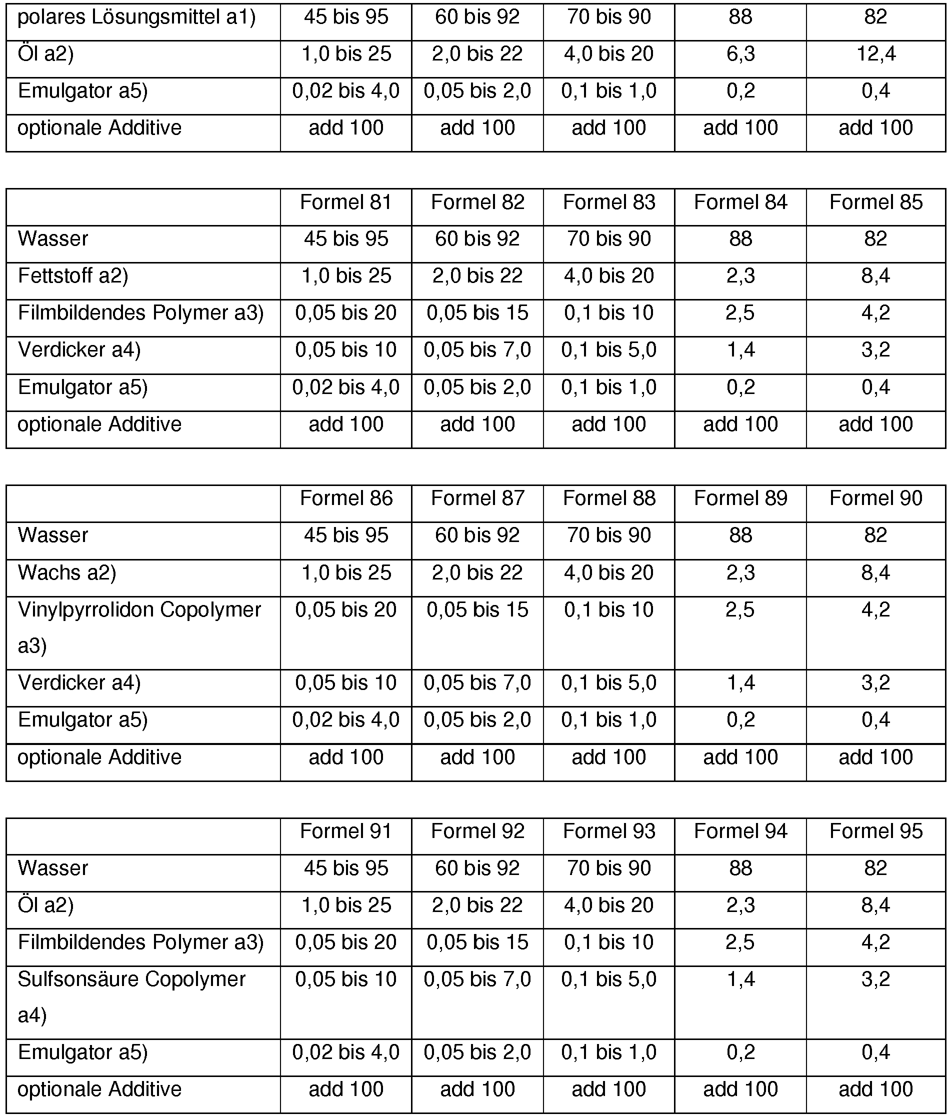

Ganz besonders bevorzugte kosmetische Zubereitungen enthalten neben den zuvor beschriebenen Bestandteilen a1) bis a5) nur geringe Mengen weiterer Wirk- und Hilfsstoffe. Kosmetische Zubereitungen, dadurch gekennzeichnet, dass der Gewichtsanteil der Bestandteile a1), a2) und, sofern vorhanden, a3), a4) und/oder a5) am Gesamtgewicht der kosmetischen Zubereitung mindestens 80 Gew.-%, vorzugsweise mindestens 87 Gew.-%, besonders bevorzugt mindestens 92 Gew.-% beträgt, sind aufgrund ihrer einfachen Herstellbarkeit und guten kosmetischen Wirkung besonders bevorzugt. Ganz besonders bevorzugte kosmetische Zubereitungen bestehen, bezogen auf ihr Gesamtgewicht, zu mindestens 80 Gew.-%, vorzugsweise mindestens 85 Gew.-% und insbesondere mindestens 87 Gew.-% aus den Bestanteilen a1), a2) und a3).

Wie eingangs ausgeführt eignen sich die erfindungsgemäßen kosmetischen Zubereitungen a) in besonderer Weise für die Applikation mittels einer Vorrichtung zur Entspannungsverdampfung. Offenbart wird die Verwendung einer kosmetischen Zubereitung a) enthaltend, bezogen auf ihr Gesamtgewicht,
a1) 45 bis 95 Gew.-% mindestens eines polaren Lösungsmittels;
a2) 1,0 bis 25 Gew.-% mindestens eines Fettstoffs;
als Prozessgut in einer Vorrichtung zur Entspannungsverdampfung.

Gegenstand der vorliegenden Anmeldung ist zudem die Verwendung eines erfindungsgemäßen Produkts zur Beaufschlagung keratinhaltiger Fasern, insbesondere menschlicher Haare, mit einer kosmetischen Zubereitung a) bzw. zur temporären Verformung keratinhaltiger Fasern, insbesondere menschlicher Haare.

Ein Verfahren zur temporären Verformung keratinhaltiger Fasern, insbesondere menschlicher Haare, bei welchem die keratinhaltigen Fasern mittels einer Vorrichtung zur Entspannungsverdampfung mit einer kosmetischen Zubereitung a) enthaltend bezogen auf ihr Gesamtgewicht
a1) 45 bis 95 Gew.-% mindestens eines polaren Lösungsmittels;
a2) 1,0 bis 25 Gew.-% mindestens eines Fettstoffs;
beaufschlagt werden, ist ein weiterer Gegenstand der vorliegenden Anmeldung. Die kosmetische Zubereitung a) wird mittels der Vorrichtung zur Entspannungsverdampfung vorzugsweise in einen Sprühnebel überführt, welcher nachfolgend die keratinhaltigen Fasern beaufschlagt.

Um eine ausreichende Sprühwirkung zu erzielen, wird die kosmetische Zubereitung a) vorzugsweise auf Temperaturen oberhalb des Siedepunktes des in der kosmetischen Zubereitung a) enthaltenen polaren Lösungsmittels oder Lösungsmittelgemisches erhitzt.

Handelt es sich bei dem polaren Lösungsmittel um Wasser oder Lösungsmittelgemische mit einem Wasseranteil oberhalb 50 Gew.-% (bezogen auf das Gesamtgewicht des Lösungsmittelgemisches), wird die kosmetische Zubereitung vorzugsweise auf Temperaturen oberhalb 100°C, vorzugsweise auf Temperaturen von 100°C und 240°C, besonders bevorzugt auf Temperaturen von 140°C bis 160°C erhitzt.

Der durch die Erhitzung der kosmetischen Zubereitung a) erzielte Überdruck beträgt in den Fällen, in denen es sich bei dem polaren Lösungsmittel um Wasser oder Lösungsmittelgemische mit einem Wasseranteil oberhalb 50 Gew.-% (bezogen auf das Gesamtgewicht des Lösungsmittelgemisches) handelt, vorzugsweise zwischen 1,1 und 8 bar, bevorzugt zwischen 1,2 und 4 bar.

Offenbart wird ein Verfahren zur Farbveränderung keratinhaltiger Fasern, insbesondere menschlicher Haare, bei welchem die keratinhaltigen Fasern mittels einer Vorrichtung zur Entspannungsverdampfung mit einer kosmetischen Zubereitung a) enthaltend bezogen auf ihr Gesamtgewicht
a1) 45 bis 95 Gew.-% mindestens eines polaren Lösungsmittels;
a2) 1,0 bis 25 Gew.-% mindestens eines Fettstoffs;
beaufschlagt werden, wobei
- aus einem Vorratsbehälter, in dessen Inneren ein Druck herrscht, der dem Umgebungsdruck entspricht, eine Teilmenge der in diesem Vorratsbehälter befindlichen kosmetischen Zubereitung a) in einen Behälter b1) überführt wird;
- nachfolgend der Zugang zwischen Vorratsbehälter und Behälter b1) durch ein Bauteil zur Durchflussregelung, mittels dessen der Durchfluss der kosmetischen Zubereitung a) aus dem Vorratsbehälter in den Behälter b1) unterbrochen werden kann, unterbrochen wird;
- nachfolgend die in dem gegen die Umgebung abgeschlossenen Behälter b1) befindliche kosmetische Zubereitung a) mittels einer Heizvorrichtung erhitzt wird, so dass der Druck im Inneren des Behälters b1) auf Werte oberhalb des Umgebungsdrucks, vorzugsweise auf Werte zwischen 1,1 und 8 bar, insbesondere auf Werte zwischen 1,2 und 4 bar ansteigt;
- nachfolgend der unter einem Druck oberhalb des Umgebungsdrucks stehende Behälter b1) in einer Weise geöffnet, wird, welche den Austritt mindestens einer Teilmenge, vorzugsweise mindestens 50 Gew.-%, bevorzugt mindestens 80 Gew.-% und insbesondere mindestens 90 Gew.-% der in dem Behälter b1) befindlichen kosmetischen Zubereitung aus dem Behälter b1) in die Umgebung unter Minderung des zum Zeitpunkt der Behälteröffnung in dem Behälter b1) herrschenden Drucks, entspannt wird.

Die Entspannung der kosmetischen Zubereitung a) in die Umgebung erfolgt vorzugsweise unter Ausbildung eines Sprühnebels der kosmetischen Zubereitung a).

Die aus dem Behälter b1) entspannte kosmetische Zubereitung a) wird vorzugsweise auf keratinische Fasern, insbesondere menschliche Haare aufgebracht.

Verfahren, in deren Verlauf die aus dem Behälter b1) entspannte kosmetische Zubereitung vor der Beaufschlagung der keratinischen Fasern durch eine Düse geleitet wird, sind besonders bevorzugt.

## Patentansprüche

1. Kosmetisches Produkt, umfassend
a) eine kosmetische Zubereitung, enthaltend bezogen auf ihr Gesamtgewicht
a1) 45 bis 95 Gew.-% mindestens eines polaren Lösungsmittels;
a2) 1,0 bis 25 Gew.-% mindestens eines Fettstoffs;
b) eine Vorrichtung zur Entspannungsverdampfung der kosmetischen Zubereitung a) mit b1
) einem Behälter, welcher einen geschlossenen Innenraum definiert, in dem die kosmetische Zubereitung aufgenommen werden kann,
b2) einem Ventil oder einem vergleichbar wirkenden Schließelement, um den wenigstens anteilsweise mit der kosmetischen Zubereitung a) befüllten Innenraum des Behälters zu verschließen und zu öffnen,
b3) einer Heizvorrichtung, um die in dem geschlossenen Innenraum des Behälters befindliche kosmetische Zubereitung a) unter Druckerhöhung zu erhitzen, sowie die erhitzte kosmetische Zubereitung a) aus dem Innenraum des Behälters unter Druckminderung in die Umgebung zu entspannen,
b4) einer Düse, welche eine Zerstäubung der aus dem Behälter entweichenden kosmetischen Zubereitung a) ermöglicht.

2. Kosmetisches Produkt nach Anspruch 1, **dadurch gekennzeichnet, dass** der Gewichtsanteil des polaren Lösungsmittels a1) am Gesamtgewicht der kosmetischen Zubereitung a) 60 bis 92 Gew.-%, vorzugsweise 70 bis 90 Gew.-% beträgt.

3. Kosmetisches Produkte nach einem der vorherigen Ansprüche, **dadurch gekennzeichnet, dass** das polare Lösungsmittel a1) ausgewählt ist aus der Gruppe Ethanol, Isopropanol, Glycerin und Wasser, vorzugsweise Wasser.

4. Kosmetisches Produkt nach einem der vorherigen Ansprüche, **dadurch gekennzeichnet, dass** der Gewichtsanteil des Fettstoffs a2) am Gesamtgewicht der kosmetischen Zubereitung a) 2,0 bis 22 Gew.-%, vorzugsweise 4,0 bis 20 Gew.-% beträgt.

5. Kosmetisches Produkt nach einem der vorherigen Ansprüche, **dadurch gekennzeichnet, dass** die kosmetische Zubereitung a) bezogen auf ihr Gesamtgewicht 0,05 bis 20 Gew.-%, vorzugsweise 0,05 bis 15 Gew.-%, und insbesondere 0,1 bis 10 Gew.-% eines filmbildenden Polymers a3) enthält.

6. Kosmetisches Produkt nach einem der vorherigen Ansprüche, **dadurch gekennzeichnet, dass** die kosmetische Zubereitung a) bezogen auf ihr Gesamtgewicht 0,05 bis 10 Gew.-%, vorzugsweise 0,05 bis 7,0 Gew.-%, und insbesondere 0,1 bis 5,0 Gew.-% eines Verdickers a4) enthält.

7. Verwendung einer kosmetischen Zubereitung a) enthaltend, bezogen auf ihr Gesamtgewicht,
a1) 45 bis 95 Gew.-% mindestens eines polaren Lösungsmittels;
a2) 1,0 bis 25 Gew.-% mindestens eines Fettstoffs;
als Prozessgut in einer Vorrichtung zur Entspannungsverdampfung der kosmetischen Zubereitung a) mit
b1) einem Behälter, welcher einen geschlossenen Innenraum definiert, in dem die kosmetische Zubereitung aufgenommen werden kann,
b2) einem Ventil oder einem vergleichbar wirkenden Schließelement, um den wenigstens anteilsweise mit der kosmetischen Zubereitung a) befüllten Innenraum des Behälters zu verschließen und zu öffnen,
b3) einer Heizvorrichtung, um die in dem geschlossenen Innenraum des Behälters befindliche kosmetische Zubereitung a) unter Druckerhöhung zu erhitzen, sowie die erhitzte kosmetische Zubereitung a) aus dem Innenraum des Behälters unter Druckminderung in die Umgebung zu entspannen,
b4) einer Düse, welche eine Zerstäubung der aus dem Behälter entweichenden kosmetischen Zubereitung a) ermöglicht.

8. Verwendung eines Produkts nach einem der Ansprüche 1 bis 6 zur Beaufschlagung keratinhaltiger Fasern, insbesondere menschlicher Haare, mit einer kosmetischen Zubereitung a).

9. Verwendung eines Produkts nach einem der Ansprüche 1 bis 6 zur temporären Verformung keratinhaltiger Fasern, insbesondere menschlicher Haare.

10. Verfahren zur temporären Verformung keratinhaltiger Fasern, insbesondere menschlicher Haare, bei welchem die keratinhaltigen Fasern mittels einer Vorrichtung zur Entspannungsverdampfung mit
b1) einem Behälter, welcher einen geschlossenen Innenraum definiert, in dem die kosmetische Zubereitung aufgenommen werden kann,
b2) einem Ventil oder einem vergleichbar wirkenden Schließelement, um den wenigstens anteilsweise mit der kosmetischen Zubereitung a) befüllten Innenraum des Behälters zu verschließen und zu öffnen,
b3) einer Heizvorrichtung, um die in dem geschlossenen Innenraum des Behälters befindliche kosmetische Zubereitung a) unter Druckerhöhung zu erhitzen, sowie die erhitzte kosmetische Zubereitung a) aus dem Innenraum des Behälters unter Druckminderung in die Umgebung zu entspannen,
b4) einer Düse, welche eine Zerstäubung der aus dem Behälter entweichenden kosmetischen Zubereitung a) ermöglicht,
mit einer kosmetischen Zubereitung a), enthaltend bezogen auf ihr Gesamtgewicht
a1) 45 bis 95 Gew.-% mindestens eines polaren Lösungsmittels;
a2) 1,0 bis 25 Gew.-% mindestens eines Fettstoffs;
beaufschlagt werden.

11. Verfahren nach Anspruch 10, **dadurch gekennzeichnet, dass**
- aus einem Vorratsbehälter, in dessen Inneren ein Druck herrscht, der dem Umgebungsdruck entspricht, eine Teilmenge der in diesem Vorratsbehälter befindlichen kosmetischen Zubereitung a) in einen Behälter überführt wird;
- nachfolgend der Zugang zwischen Vorratsbehälter und Behälter durch ein Bauteil zur Durchflussregelung, mittels dessen der Durchfluss der kosmetischen Zubereitung a) aus dem Vorratsbehälter in den Behälter unterbrochen werden kann, unterbrochen wird;
- nachfolgend die in dem gegen die Umgebung abgeschlossenen Behälter befindliche kosmetische Zubereitung a) mittels einer Heizvorrichtung erhitzt wird, so dass der Druck im Inneren des Behälters auf Werte oberhalb des Umgebungsdrucks, vorzugsweise auf Werte zwischen 1,1 und 8 bar, insbesondere auf Werte zwischen 1,2 und 4 bar ansteigt;
- nachfolgend der unter einem Druck oberhalb des Umgebungsdrucks stehende Behälter in einer Weise geöffnet, wird, welche den Austritt mindestens einer Teilmenge, vorzugsweise mindestens 50 Gew.-%, bevorzugt mindestens 80 Gew.-% und insbesondere mindestens 90 Gew.-% der in dem Behälter befindlichen kosmetischen Zubereitung aus dem Behälter in die Umgebung unter Minderung des zum Zeitpunkt der Behälteröffnung in dem Behälter herrschenden Drucks, entspannt wird.

## Claims

1. A cosmetic product, comprising
a) a cosmetic preparation, containing, based on its total weight,
a1) 45 to 95 wt.% at least one polar solvent;
a2) 1.0 to 25 wt.% at least one fatty substance;
b) a device for flash evaporation of the cosmetic preparation a), comprising
b1) a container, which defines a closed inner chamber in which the cosmetic preparation can be stored,
b2) a valve or a similarly functioning closing element, in order to close and open the inner chamber of the container filled at least in part with the cosmetic preparation a),
b3) a heating device, in order to heat the cosmetic preparation a) in the closed inner chamber of the container by increasing the pressure, and to pressure-relieve the heated cosmetic preparation a) from the inner chamber of the container into the surrounding region by lowering the pressure,
b4) a nozzle, which makes it possible to atomize the cosmetic preparation a) escaping from the container.

2. The cosmetic product according to claim 1, **characterized in that** the weight proportion of the polar solvent a1) with respect to the total weight of the cosmetic preparation a) is 60 to 92 wt.%, preferably 70 to 90 wt.%.

3. The cosmetic product according to one of the preceding claims, **characterized in that** the polar solvent a1) is selected from the group comprising ethanol, isopropanol, glycerol and water, preferably water.

4. The cosmetic product according to one of the preceding claims, **characterized in that** the weight proportion of the fatty substance a2) with respect to the total weight of the cosmetic preparation a) is 2.0 to 22 wt.%, preferably 4.0 to 20 wt.%.

5. The cosmetic product according to one of the preceding claims, **characterized in that** the cosmetic preparation a) contains, based on its total weight, 0.05 to 20 wt.%, preferably 0.05 to 15 wt.%, and in particular 0.1 to 10 wt.%, a film-forming polymer a3).

6. The cosmetic product according to one of the preceding claims, **characterized in that** the cosmetic preparation a) contains, based on its total weight, 0.05 to 10 wt.%, preferably 0.05 to 7.0 wt.%, and in particular 0.1 to 5.0 wt.%, a thickener a4).

7. The use of a cosmetic preparation a) containing, based on its total weight,
a1) 45 to 95 wt.% at least one polar solvent;
a2) 1.0 to 25 wt.% at least one fatty substance;
as a process material in a device for flash evaporation of the cosmetic preparation a), comprising
b1) a container, which defines a closed inner chamber in which the cosmetic preparation can be stored,
b2) a valve or a similarly functioning closing element, in order to close and open the inner chamber of the container filled at least in part with the cosmetic preparation a),
b3) a heating device, in order to heat the cosmetic preparation a) in the closed inner chamber of the container by increasing the pressure, and to pressure-relieve the heated cosmetic preparation a) from the inner chamber of the container into the surrounding region by lowering the pressure,
b4) a nozzle, which makes it possible to atomize the cosmetic preparation a) escaping from the container.

8. The use of a product according to one of claims 1 to 6 for subjecting keratin-containing fibers, in particular human hair, to a cosmetic preparation a).

9. The use of a product according to one of claims 1 to 6 for temporarily reshaping keratin-containing fibers, in particular human hair.

10. A method for temporarily reshaping keratin-containing fibers, in particular human hair, in which the keratin-containing fibers are subjected, by means of a device for flash evaporation, comprising
b1) a container, which defines a closed inner chamber in which the cosmetic preparation can be stored,
b2) a valve or a similarly functioning closing element, in order to close and open the inner chamber of the container filled at least in part with the cosmetic preparation a),
b3) a heating device, in order to heat the cosmetic preparation a) in the closed inner chamber of the container by increasing the pressure, and to pressure-relieve the heated cosmetic preparation a) from the inner chamber of the container into the surrounding region by lowering the pressure,
b4) a nozzle, which makes it possible to atomize the cosmetic preparation a) escaping from the container,
to a cosmetic preparation a), containing, based on its total weight,
a1) 45 to 95 wt.% at least one polar solvent;
a2) 1.0 to 25 wt.% at least one fatty substance.

11. The method according to claim 10, **characterized in that**
- a portion of the cosmetic preparation a) in a reservoir, inside which there is a pressure corresponding to the ambient pressure, is transferred out of said reservoir into a container;
- the access between the reservoir and the container is subsequently disrupted by a component for controlling flow, by means of which component the flow of the cosmetic preparation a) out of the reservoir into the container can be disrupted;
- the cosmetic preparation a) in the container, which is sealed against the surrounding region, is subsequently heated by means of a heating device, such that the pressure inside the container increases to values above the ambient pressure, preferably to values between 1.1 and 8 bar, in particular to values between 1.2 and 4 bar;
- the container under pressure that is above the ambient pressure is subsequently opened in such a way that the discharge of at least one portion, preferably at least 50 wt.%, more preferably at least 80 wt.% and in particular at least 90 wt.%, of the cosmetic preparation in the container is pressure-relieved from the container into the surrounding region by the pressure prevailing in the container at the time at which the container is opened being reduced.

## Revendications

1. Produit cosmétique comprenant
a) une préparation cosmétique contenant, sur la base de son poids total,
a1) de 45 à 95 % en poids d'au moins un solvant polaire ;
a2) de 1,0 à 25 % en poids d'au moins un corps gras ;
b) un dispositif de vaporisation éclair de la préparation cosmétique a) comprenant
b1) un récipient qui définit un espace intérieur fermé dans lequel la préparation cosmétique peut être reçue,
b2) une soupape ou un élément de fermeture à action comparable pour fermer et ouvrir l'espace intérieur du récipient rempli au moins en partie avec la préparation cosmétique a),
b3) un dispositif de chauffage destiné à chauffer la préparation cosmétique a) située dans l'espace intérieur fermé du récipient par augmentation de la pression, ainsi que pour détendre la préparation cosmétique chauffée a) de l'espace intérieur du récipient dans l'environnement par réduction de la pression,
b4) une buse qui permet l'atomisation de la préparation cosmétique s'échappant du récipient a).

2. Produit cosmétique selon la revendication 1, **caractérisé en ce que** la proportion en poids du solvant polaire a1) sur le poids total de la préparation cosmétique a) est de 60 à 92 % en poids, de préférence de 70 à 90 % en poids.

3. Produit cosmétique selon l'une des revendications précédentes, **caractérisé en ce que** le solvant polaire a1) est choisi dans le groupe comprenant l'éthanol, l'isopropanol, le glycérol et l'eau, de préférence l'eau.

4. Produit cosmétique selon l'une des revendications précédentes, **caractérisé en ce que** la proportion en poids du corps gras a2) sur le poids total de la préparation cosmétique a) est de 2,0 à 22 % en poids, de préférence de 4,0 à 20 % en poids.

5. Produit cosmétique selon l'une des revendications précédentes, **caractérisé en ce que** la préparation cosmétique a) contient, sur la base de son poids total, de 0,05 à 20 % en poids, de préférence de 0,05 à 15 % en poids, et en particulier de 0,1 à 10 % en poids d'un polymère filmogène a3).

6. Produit cosmétique selon l'une des revendications précédentes, **caractérisé en ce que** la préparation cosmétique a) contient, sur la base de son poids total, de 0,05 à 10 % en poids, de préférence de 0,05 à 7,0 % en poids, et en particulier de 0,1 à 5,0 % en poids d'un épaississant a4).

7. Utilisation d'une préparation cosmétique a) contenant, sur la base de son poids total,
a1) de 45 à 95 % en poids d'au moins un solvant polaire ;
a2) de 1,0 à 25 % en poids d'au moins un corps gras ;
comme matière de processus dans un dispositif de vaporisation éclair de la préparation cosmétique a) avec
b1) un récipient qui définit un espace intérieur fermé dans lequel la préparation cosmétique peut être reçue,
b2) une soupape ou un élément de fermeture à action comparable pour fermer et ouvrir l'espace intérieur du récipient rempli au moins en partie avec la préparation cosmétique a),
b3) un dispositif de chauffage destiné à chauffer la préparation cosmétique a) située dans l'espace intérieur fermé du récipient par augmentation de la pression, ainsi que pour détendre la préparation cosmétique chauffée a) de l'espace intérieur du récipient dans l'environnement par réduction de la pression,
b4) une buse qui permet l'atomisation de la préparation cosmétique s'échappant du récipient a).

8. Utilisation d'un produit selon l'une des revendications 1 à 6 pour soumettre des fibres de kératine, en particulier des cheveux humains, à une préparation cosmétique a).

9. Utilisation d'un produit selon l'une des revendications 1 à 6 pour la mise en forme temporaire des fibres de kératine, en particulier de cheveux humains.

10. Procédé de mise en forme temporaire de fibres de kératines, en particulier de cheveux humains, dans lequel les fibres de kératine sont soumises, au moyen d'un dispositif de vaporisation éclair comprenant
b1) un récipient qui définit un espace intérieur fermé dans lequel la préparation cosmétique peut être reçue,
b2) une soupape ou un élément de fermeture à action comparable pour fermer et ouvrir l'espace intérieur du récipient rempli au moins en partie avec la préparation cosmétique a),
b3) un dispositif de chauffage destiné à chauffer la préparation cosmétique a) située dans l'espace intérieur fermé du récipient par augmentation de la pression, ainsi que pour détendre la préparation cosmétique chauffée a) de l'espace intérieur du récipient dans l'environnement par réduction de la pression,
b4) une buse qui permet l'atomisation de la préparation cosmétique s'échappant du récipient a),
à une préparation cosmétique a) contenant, sur la base de son poids total,
a1) de 45 à 95 % en poids d'au moins un solvant polaire ;
a2) 1,0 à 25 % en poids d'au moins un corps gras.

11. Procédé selon la revendication 10, **caractérisé en ce que**
- à partir d'un récipient de stockage, à l'intérieur duquel règne une pression qui correspond à la pression ambiante, une partie de la préparation cosmétique a) se trouvant dans ce récipient de stockage est transférée dans un récipient ;
- puis l'accès entre le récipient de stockage et le récipient est interrompu par un composant de régulation de débit permettant d'interrompre l'écoulement de la préparation cosmétique a) du récipient de stockage dans le récipient ;
- puis la préparation cosmétique a) se trouvant dans le récipient isolé de l'environnement est chauffée au moyen d'un dispositif de chauffage de sorte que la pression à l'intérieur du récipient monte à des valeurs au-dessus de la pression ambiante, de préférence à des valeurs comprises entre 1,1 et 8 bars, notamment à des valeurs comprises entre 1,2 et 4 bars ;
- puis le récipient à une pression supérieure à la pression ambiante est ouvert de manière à générer une détente qui fait passer au moins une partie, avantageusement au moins 50 % en poids, de préférence au moins 80 % en poids et en particulier au moins 90 % en poids de la préparation cosmétique se trouvant dans le récipient, du récipient dans l'environnement tout en réduisant la pression régnant dans le récipient au moment de l'ouverture du récipient.
